# EUROPEAN PATENT APPLICATION

(11) **EP 4 550 335 A1**
(43) Date of publication of application: **07.05.2025**
(21) Application number: 24210286.1
(22) Date of filing: 31.10.2024
(51) Int. Cl.: G16B 35/00, G16B 20/00, G16B 30/00, G16B 40/00

(54) **T CELL RECEPTOR SCREENING METHODS**

(30) Priority: 31.10.2023 US 202363594857 P
(71) Applicant: Tempus AI, Inc., Chicago, IL 60654 (US)
(72) Inventor: FIELDS, Paul, Chicago, 60654 (US); HARDING, Taylor, Chicago, 60654 (US); KHAN, Aly A., Chicago, 60654 (US); LUO, Fu, Chicago, 60654 (US); RAND, Timothy, Chicago, 60654 (US); ROSASCO, Mario G., Chicago, 60654 (US); STEIN, Michelle M., Chicago, 60654 (US); VASQUEZ OSPINA, Juan Jose, Chicago, 60654 (US)
(74) Representative: Grund, Martin

(57) **Abstract**

TCR identification methods that find particular use in the development of TCR-based cancer therapies are described. The subject TCR screening methods utilize a comprehensive cancer subject database to effectively identify candidate TCRs that recognize a peptide of interest (e.g., a neoantigen) in complex with an HLA allele.

## Description

### RELATED APPLICATIONS

This application claims priority to U.S. Provisional Application No. 63/594,857, filed on October 31, 2023, and is entitled "T CELL RECEPTOR SCREENING METHODS," which is hereby incorporated by reference in its entirety.

### BACKGROUND

T cell receptor-based therapies rely on the use of engineered TCRs capable of recognizing peptide/MHC (pMHC) complexes. TCR-based therapies utilize such engineered TCRs to activate the immune system (adoptive transfer therapies), or to localize immune cells to tumors (e.g., ImmTAC (immune mobilizing monoclonal TCRs against cancer)). Examples of peptide epitopes include those derived from tumor-associated antigens, and neoantigens that are caused, for example, by gene mutations in tumors during carcinogenesis.

While T cell receptor-based therapies have shown promising preliminary results, the development of such therapies have been limited by the ability to effectively and efficiently screen for TCRs that recognize peptide/MHC complexes. Current screening methods generally depend on a mass screening approach, wherein a large recombinant TCR library is constructed (e.g., 10¹⁵ - 10¹⁶ potential TCR combinations) and used to identify individual TCRs that bind to a peptide epitope derived from an antigen of interest. Such screens are labor-intensive, inefficient and costly. Thus, there is a need for new TCR screening methods for use in the development of TCR-based cancer therapies.

### SUMMARY

Provided herein, inter alia, is a novel TCR screening method that finds particular use in target selection for the development of TCR-based therapies, including cancer therapies. In embodiments, the subject TCR screening method utilizes a comprehensive cancer subject database to effectively identify candidate TCRs that bind to a peptide of interest (e.g., a neoepitope derived from a neoantigen) in complex with an HLA molecule associated with a particular HLA allele.

In one aspect, provided herein is a method of identifying a T cell receptor (TCR) that is capable of binding to a peptide of interest in complex with a human leukocyte antigen (HLA). The method comprises at a computer system having one or more processors, and memory storing one or more programs for execution by the one or more processors: a) identifying a first plurality of sequences for a first T-Cell Receptor (TCR) chain and a second plurality of sequences for a second TCR chain from a plurality of sequence read profiles, wherein each respective sequence read profile in the plurality of sequence read profiles comprises corresponding sequence reads for amino acids in a biological sample from a respective subject in a plurality of subjects, and wherein the first TCR chain and second TCR chain are a TCR α-chain and a TCR-β-chain, respectively, or a TCR γ-chain and a TCR δ-chain, respectively; b) clustering the first plurality of sequences into a first plurality of sequence groups based on sequence similarity between the respective sequences for the first TCR chain in the first plurality of sequences; c) clustering the second plurality of sequences into a second plurality of sequence groups based on sequence similarity between the respective sequences for the second TCR chain in the second plurality of sequences; d) identifying a first subset of the first plurality of sequence groups containing sequences that are more prevalent in a first subset of the plurality of subjects that have both the peptide of interest and the HLA than in a second subset of the plurality of subjects that do not have both the peptide of interest and the HLA; e) identifying a second subset of the second plurality of sequence groups containing sequences that are more prevalent in the first subset of the plurality of subjects that have the peptide of interest and the HLA than in the second subset of the plurality of subjects that do not have the peptide of interest and the HLA; f) identifying a plurality of TCR candidate pairs, wherein each respective TCR candidate pair comprises a respective sequence for the first TCR chain present in the first subset of the first plurality of sequence groups or a variant thereof and a respective sequence for the second TCR chain present in the second subset of the second plurality of sequence groups or a variant thereof, and g) screening the plurality of TCR candidate pairs for the ability to bind the peptide of interest, thereby identifying a T cell receptor (TCR) that is capable of binding to a peptide of interest.

In some embodiments, the first TCR chain is a TCR α-chain and the second TCR chain is a TCR β-chain. In some embodiments, the first TCR chain is a TCR γ-chain and the second TCR chain is a TCR δ-chain. In certain embodiments, the plurality of sequence read profiles comprises at least 500, at least 1,000, at least 5,000, at least 10,000, at least 20,000, at least 50,000, at least 100,000, at least 500,000, at least 1 million, at least 5 million, or at least 10 million sequence read profiles.

In some embodiments, the corresponding sequence reads for the amino acids in the biological sample from the respective subject are sequence reads from a cancerous tissue of the respective subject. In exemplary embodiments, the plurality of subjects comprises cancer subjects. In some embodiments, the cancer is selected from urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer.

In some embodiments, the peptide of interest is an epitope derived from a tumor-associated antigen, a tumor-specific antigen, or a neoantigen. In some embodiments, the cancer subjects have undergone a treatment for the cancer. In exemplary embodiments, the treatment is selected from surgery, chemotherapy, immunotherapy, bone marrow transplant, immunotherapy, hormone therapy, targeted drug therapy, cryoablation, radiofrequency ablation, and combinations thereof. In some embodiments, the immunotherapy is selected from an antibody therapy, a cytokine therapy, an oncolytic virus therapy, an adoptive cell transplant therapy, a cancer vaccine or combinations thereof.

In some embodiments, the immunotherapy is an antibody therapy. In some embodiments, the antibody therapy is a human checkpoint inhibitor therapy, and wherein the human checkpoint is selected from one of the following: PD-1, PD-L1, CTLA-4, LAG3, TIM-3, B7H3, B7H4, A2aR, CD73, NIKG2A, PVRIG/PVRL2, CEACAM1, CECAM 5/6, FAK, CCL2/CCR2, LIF, CD47/SIRPα, CSF-1, IL-1, IL-1R3, IL-8, SEMA4D, Ang-2, CLEVER-1, Axl, and phosphatidylserine.

In some embodiments, the plurality of subjects do not exhibit tumor cell loss of heterozygosity.

In some embodiments, the peptide of interest is derived from a tumor neoantigen selected from KRAS:p.G12D, BRAF:p.V600E, KRAS:p.G12V, ACVR2A:p.K435fs, GRB14:p.KKK295del, SEC63:p.L532fs, TGFBR2:p.E125fs, ATR:p.K771fs, ICAl:p.N204fs, KRAS:p.G12C, TP53:p.R175H, ABCA8:p.R842Q, ACTL7B:p.R354H, ACVR2A:p.K435fs, AIM2:p.K340fs, ALG2:p.S302Y, ANKIB 1 :p.K144fs, ARSG:p.V131I, ATP10D:p.R311H, AXIN2:p.W663fs, C5orf30:p.D4N, CACNG3:p.V134I, CASP5:p.K78fs, CC2D2A:p.R1284C, CDH10:p.E349K, DNMT1:p.E432K, DOCK2:p.G170R, DOCKS:p.E177K, EGR2:p.R390H, ERBB3:p.V104M, FAM135B:p.R884H, FBXW7:p.R505C, FBXW7:p.R465H, FHDC1:p.R254W, FOXL1:p.N89K, HCN4:p.R525H, HLA-DMA:p.E84K, HTR3B:p.R236C, ITGA4:p.T673M, KIF18A:p.R17C, KIF20B:p.E991K, KLHL5:p.R326C, KRAS:p.A146T, KRAS:p.G13D, LPHN3:p.R1183Q, MAP2K4:p.R287H, MAPK8IP1:p.L217fs, MFSD5:p.R280Q, MUC16:p.R8606H, MYO6:p.D1180N, NAA25:p.S807Y, NBPF14:p.V44L, NRAS:p.Q61K, NRAS:p.G13R, PAX3:p.T424M, PGAM1:p.R240H, PHF3:p.R1410I, PIK3CA:p.R88Q, PIK3CA:p.E545K, PIK3CA:p.H1047R, PLXNA3:p.V14fs, POSTN:p.R508C, PTPRU:p.D1434N, PYGo2:p.Q150fs, RBBP7:p.E274K, SFPQ:p.R611Q, SGSM1:p.F1117L, SLC25A40:p.R96Q, SLC8A1:p.R431H, SLITRK3:p.S298L, SPATA22:p.S150L, SUN3:p.E128K, TGFBR1:p.S241L, TP53:p.R273H, TP53:p.R273C, TP53:p.R248W, TRPVS:p.R492H, USP40:p.S851L, VPS13C:p.D1359Y, ZBTB24:p.L607I, ZNF434:p.R306C, ZNF443:p.R301I, ZNF484:p.R138C, and ZNF770:p.S441P. In certain embodiments, the tumor neoantigen and HLA allele are selected from the following neoantigen and HLA allele pairs: TP53 (R175H)-A*02:01, TP53 (Y220C)-A*02:01, and TP53 (R248W)-A*68:01.

In some embodiments, the plurality of subjects comprises subjects having a viral infection, and wherein the peptide of interest is an epitope derived from an antigen of the virus.

In some embodiments, the clustering of the first plurality of sequences and/or the clustering of the second plurality of sequences is based on at least CDR3 sequence similarity. In exemplary embodiments, the clustering of the first plurality of sequences and/or the clustering of the second plurality of sequences is based on at least CDR3 length. In exemplary embodiments, the clustering of the first plurality of sequences and/or the clustering of the second plurality of sequences is based on identical sequence length and sequence similarity. In some embodiments, the clustering of the first plurality of sequences and/or the clustering of the second plurality of sequence is performed on at least 1 × 10², at least 1 × 10³, at least 1 × 10⁴, at least 1 × 10⁵, or at least 1 × 10⁶ sequences.

In some embodiments, the respective sequences for the first TCR chain in the first plurality of sequence groups and the respective sequences for the second TCR chain in the second plurality of sequence groups are collectively representative of TCR sequences in the corresponding biological samples for at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95% or at least 99% of the plurality of subjects.

In some embodiments, the first subset of the first plurality of sequence groups contains TCR α-chain sequences and the second subset of the second plurality of sequence groups contains TCR β-chain sequences. In some embodiments, the first subset of the first plurality of sequence groups contains TCR γ-chain sequences and the second subset of the second plurality of sequence groups contains TCR δ-chain sequences.

In exemplary embodiments, the sequences of the first plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects. In exemplary embodiments, sequences of the second plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects.

In some embodiments, the first threshold percentage is about 0.1%, about 0.5%, about 1%, about 5%, about 10%, about 15%, about 20%, about 25%, about 30%, about 35%, about 40%, about 45%, about 50%, about 60%, about 65%, about 70%, about 75%, about 80%, about 85%, about 90%, about 95% or about 99%. In certain embodiments, the second threshold percentage is about 0.5%, about 1%, about 5%, about 10%, about 15%, about 20%, about 25%, about 30%, about 35%, about 40%, about 45%, or about 50%.

In some embodiments, respective subjects in the first subset of the plurality of subjects and the second subset of the plurality of subjects are identified based on sequencing data from corresponding samples of cancerous tissues from the respective subjects.

In some embodiments, the identifying f) comprises screening for TCR pairs in which the respective sequence for the first TCR chain and the respective sequence for the second TCR chain are co-expressed in a threshold number of subjects in the first subset of the plurality of subjects. In some embodiments, the threshold number of subjects is at least 10, at least 25, at least 50, or at least 100 subjects.

In some embodiments, the respective sequence for the first TCR chain comprises a CDR3 sequence of the first TCR chain and the respective sequence for the second TCR chain comprises a CDR3 sequence of the second TCR chain.

In some embodiments, each respective TCR candidate pair comprises a first TCR chain and a second TCR chain that is expressed in the same subject. In some embodiments, first TCR chain is a TCR α-chain and the second TCR chain is a TCR β-chain. In some embodiments, the first TCR chain is a TCR γ-chain and the second TCR chain is a TCR δ-chain.

In some embodiments, the screening step g) comprises expressing the plurality of TCR candidate pairs in a plurality of cells, wherein an individual cell of the plurality of cells express a TCR candidate pair of the plurality of TCR candidate pairs. In some embodiments, the screening step g) comprises contacting the plurality of cells expressing the plurality of TCR candidate pairs with peptide of interest/MHC (pMHC) multimers and isolating cells that bind the pMHC multimers. In exemplary embodiments, the cells that bind the pMHC multimers are isolated using a cell sorting method. In some embodiments, the cell sorting method is selected from magnetic-activated cell sorting (MACS), fluorescence-activated cell sorting (FACS), and buoyancy-activated cell sorting. In some embodiments, the pMHC multimers are tetramers.

In some embodiments, one or more TCR candidate pairs in the plurality of TCR candidate pairs comprises: a) a variant of a TCR α-chain that is present in the first subset of the first plurality of sequence groups; and/or b) variant of a first TCR β -chain that is present in the second subset of the second plurality of sequence groups.

In some embodiments, the variant TCR α-chain has the CDR3 sequence of a TCR α-chain that is present in the first subset of the first plurality of sequence groups. In some embodiments, the variant TCR α-chain does not have the CDR1 sequence and/or CDR2 sequence of the TCR α-chain that is present in the first subset of the first plurality of sequence groups. In exemplary embodiments, the variant TCR β -chain has the CDR3 sequence of a TCR β -chain that is present in the second subset of the second plurality of sequence groups. In some embodiments, the variant β-chain does not have the CDR1 sequence and/or CDR2 sequence of the TCR β -chain that is present in the second subset of the second plurality of sequence groups.

In some embodiments, the variant TCR α-chain is at least about 70%, at least about 80%, at least about 90%, at least about 95%, or at least about 98% identical to the TCR α-chain that is present in the first subset of the first plurality of sequence groups.

In some embodiments, the variant TCR β-chain is at least about 70%, at least about 80%, at least about 90%, at least about 95%, or at least about 98% identical to the TCR β-chain that is present in the second subset of the second plurality of sequence groups.

In some embodiments, one or more TCR candidate pairs of the plurality of TCR candidate pairs comprises: a) a variant of a TCR γ-chain that is present in the first subset of the first plurality of sequence groups; and/or b) variant of a first TCR δ -chain that is present in the second subset of the second plurality of sequence groups.

In some embodiments, the variant TCR γ-chain has the CDR3 sequence of a TCR α-chain that is present in the first subset of the first plurality of sequence groups. In some embodiments, the variant TCR γ-chain does not have the CDR1 sequence and/or CDR2 sequence of the TCR γ-chain that is present in the first subset of the first plurality of sequence groups. In some embodiments, the variant TCR δ-chain has the CDR3 sequence of a TCR β - chain that is present in the second subset of the second plurality of sequence groups. In some embodiments, the variant δ-chain does not have the CDR1 sequence and/or CDR2 sequence of the TCR δ -chain that is present in the second subset of the second plurality of sequence groups. In some embodiments, the variant TCR γ-chain is at least about 70%, at least about 80%, at least about 90%, at least about 95%, or at least about 98% identical to the TCR γ-chain that is present in the first subset of the first plurality of sequence groups. In some embodiments, the variant TCR δ -chain is at least about 70%, at least about 80%, at least about 90%, at least about 95%, or at least about 98% identical to the TCR δ-chain that is present in the second subset of the second plurality of sequence groups.

In exemplary embodiments, the method further comprises sequencing candidate TCRs that are determined to bind to a peptide of interest in complex with the HLA.

In some embodiments, the screening g) comprises in silico modeling of an interaction between respective TCR candidate pairs and the peptide of interest.

In another aspect, provided herein is a computer system comprising: one or more processors; and a non-transitory computer-readable medium including computer-executable instructions that, when executed by the one or more processors, cause the processors to perform the method provided herein.

In another aspect, provided herein is a non-transitory computer-readable storage medium having stored thereon program code instructions that, when executed by a processor, cause the processor to perform the subject method provided herein.

In one aspect, provided herein is a method of identifying a T cell receptor (TCR) that is capable of binding to a peptide of interest in complex with a human leukocyte antigen (HLA) encoded by a particular HLA allele. In this method, enriched TCR α-chains and TCR-β-chains and/or TCR γ-chains and TCR δ-chains are first identified in a first group of subjects that express the peptide of interest and HLA from a plurality of subjects using a subject profile database (step a)). The subject profile database comprises a subject profile for each individual subject in the plurality of subjects, wherein each subject profile comprises: i) a TCR profile of TCR α-chains and TCR β-chains and/or TCR γ-chains and TCR δ-chains of a subject; ii) an HLA allele profile of HLA alleles of a subject; and iii) a peptide of interest profile comprising information relating to the presence or amount of a peptide of interest in a subject. Next, a TCR library is prepared (step b)), the TCR library comprising a first plurality of polynucleotides and a second plurality of polynucleotides, wherein each of the polynucleotides in the first plurality of polynucleotides encodes for an enriched TCR α-chain or TCR γ-chain identified in step a) or a variant thereof, and wherein each of the polynucleotides in the second plurality of polynucleotides encodes for an enriched TCR β-chain or TCR δ-chain identified in step a) or variant thereof. The TCR library is then expressed to form a plurality of candidate TCRs (step c)), where each of the candidate TCRs in the plurality comprise an enriched TCR α-chain and β-chain and/or TCR γ-chain and TCR δ-chain identified in step a) or a variant thereof. Further, the ability of candidate TCRs from the plurality of candidate TCRs to bind to the peptide of interest in complex with the HLA is assessed (step d)).

In some embodiments, the identifying step a) comprises selecting a first group of subject profiles that include the peptide of interest and HLA, and determining TCR α-chain, β-chain, γ-chain and and/or δ-chain sequences present in the subject profiles of the first group of subject profiles. In certain embodiments, the identifying step a) further comprises classifying a TCR α-chain, β-chain, γ-chain and and/or δ-chain sequence present in the subject profiles of the first group of subject profiles as enriched in the first group of subjects if the TCR α-chain, β-chain, γ-chain and and/or δ-chain sequence is present above a threshold percentage of the subject profiles in the first group of subject profiles. In embodiments, the threshold percentage is about 0.1%, about 0.5%, about 1%, about 5%, about 10%, about 15%, about 20%, about 25%, about 30%, about 35%, about 40%, about 45%, about 50%, about 60%, about 65%, about 70%, about 75%, about 80%, about 85%, about 90%, about 95% or about 99%.

In some embodiments, the identifying step a) further comprises selecting a second group of patient profiles that include the HLA allele and do not include the peptide of interest, and determining TCR α-chain, β-chain, γ-chain and and/or δ-chain sequences present in the second group of patient profiles. In some embodiments, the identifying step a) further comprises classifying a TCR α-chain, β-chain, γ-chain and and/or δ-chain sequence present in the patient profiles of the first group of patient profile as enriched in the first group of cancer patients if the TCR α-chain, β-chain, γ-chain and and/or δ-chain sequence is present above a first threshold percentage of the patient profiles in the first group of patient profiles and below a second threshold percentage in the patient profiles of the second group of patient profiles. In embodiments, the first threshold percentage is 0.1%, about 0.5%, about 1%, about 5%, about 10%, about 15%, about 20%, about 25%, about 30%, about 35%, about 40%, about 45%, about 50%, about 60%, about 65%, about 70%, about 75%, about 80%, about 85%, about 90%, about 95% or about 99%. In certain embodiments, the second threshold percentage is about 0.5%, about 1%, about 5%, about 10%, about 15%, about 20%, about 25%, about 30%, about 35%, about 40%, about 45%, or about 50%.

In embodiments, a cluster analysis is performed on the TCR α-chain and TCR β-chain sequences present in the patient profiles of the first group of patient profiles prior to the classifying, wherein the cluster analysis clusters the TCR α-chain and TCR β-chain sequences based on sequence similarity. In some embodiments, a cluster analysis is performed on the TCR α-chain and TCR β-chain sequences present in the patient profiles of the first and second groups of patient profiles prior to the classifying, wherein the cluster analysis clusters the TCR α-chain and TCR β-chain sequences based on sequence similarity. In embodiments, the clustering analysis is performed on at least about 1 × 10², at least about 1 × 10³, at least about 1 × 10⁴, at least about 1 × 10⁵, or at least about 1 × 10⁶ TCR α-chain and TCR β-chain sequences.

In some embodiments, a cluster analysis is performed on the TCR γ-chain and TCR δ-chain sequences present in the patient profiles of the first group of patient profiles prior to the classifying, wherein the cluster analysis clusters the TCR γ-chain and TCR δ-chain sequences based on sequence similarity. In some embodiments, a cluster analysis is performed on the TCR γ-chain and TCR δ-chain sequences present in the patient profiles of the first and second groups of patient profiles prior to the classifying, wherein the cluster analysis clusters the TCR γ-chain and TCR δ-chain chain sequences based on sequence similarity. In some embodiments, the clustering analysis is performed on at least about 1 × 10², at least about 1 × 10³, at least about 1 × 10⁴, at least about 1 × 10⁵, or at least about 1 × 10⁶ TCR γ-chain and TCR δ-chain sequences.

In embodiments of the method, the sequence similarity comprises CDR3 sequence similarity.

In some embodiments, the subject profile database comprises at least about 500, at least about 1,000, at least about 5,000, at least about 10,000, at least about 20,000, at least about 50,000, or at least about 100,000 subject profiles.

In embodiments, the TCR profile comprises a profile of TCR α-chains and TCR β-chains of a subject. In embodiments, the TCR data comprises a profile of TCR γ-chain and TCR δ-chain of a subject.

In some embodiments, the plurality of subjects are cancer patients that each have the same cancer type, and the peptide of interest is an epitope derived from a tumor-associated antigen, a tumor-specific antigen, or a neoantigen. In some embodiments, the cancer type is selected from urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer.

In some embodiments, the plurality of subjects comprises subjects who have undergone a treatment for the cancer. In exemplary embodiments, the treatment is selected from surgery, chemotherapy, immunotherapy, bone marrow transplant, immunotherapy, hormone therapy, targeted drug therapy, cryoablation, radiofrequency ablation, and combinations thereof. In some embodiments, the immunotherapy is selected from an antibody therapy, a cytokine therapy, an oncolytic virus therapy, an adoptive cell transplant therapy, a cancer vaccine or combinations thereof.

In certain embodiments, the immunotherapy is an antibody therapy. In some embodiments, the antibody therapy is a human checkpoint inhibitor therapy, and wherein the human checkpoint is selected from one of the following: PD-1, PD-L1, CTLA-4, LAG3, TIM-3, B7H3, B7H4, A2aR, CD73, NIKG2A, PVRIG/PVRL2, CEACAM1, CECAM 5/6, FAK, CCL2/CCR2, LIF, CD47/SIRPα, CSF-1, IL-1, IL-1R3, IL-8, SEMA4D, Ang-2, CLEVER-1, Axl, and phosphatidylserine.

In some embodiments, the plurality of subjects do not exhibit tumor cell loss of heterozygosity.

In some embodiments, the peptide of interest is derived from a tumor neoantigen selected from KRAS:p.G12D, BRAF:p.V600E, KRAS:p.G12V, ACVR2A:p.K435fs, GRB14:p.KKK295del, SEC63:p.L532fs, TGFBR2:p.E125fs, ATR:p.K771fs, ICAl:p.N204fs, KRAS:p.G12C, TP53:p.R175H, ABCA8:p.R842Q, ACTL7B:p.R354H, ACVR2A:p.K435fs, AIM2:p.K340fs, ALG2:p.S302Y, ANKIB 1 :p.K144fs, ARSG:p.V131I, ATP10D:p.R311H, AXIN2:p.W663fs, C5orf30:p.D4N, CACNG3:p.V134I, CASP5:p.K78fs, CC2D2A:p.R1284C, CDH10:p.E349K, DNMT1:p.E432K, DOCK2:p.G170R, DOCKS:p.E177K, EGR2:p.R390H, ERBB3:p.V104M, FAM135B:p.R884H, FBXW7:p.R505C, FBXW7:p.R465H, FHDC1:p.R254W, FOXL1:p.N89K, HCN4:p.R525H, HLA-DMA:p.E84K, HTR3B:p.R236C, ITGA4:p.T673M, KIF18A:p.R17C, KIF20B:p.E991K, KLHL5:p.R326C, KRAS:p.A146T, KRAS:p.G13D, LPHN3:p.R1183Q, MAP2K4:p.R287H, MAPK8IP1:p.L217fs, MFSD5:p.R280Q, MUC16:p.R8606H, MYO6:p.D1180N, NAA25:p.S807Y, NBPF14:p.V44L, NRAS:p.Q61K, NRAS:p.G13R, PAX3:p.T424M, PGAM1:p.R240H, PHF3:p.R1410I, PIK3CA:p.R88Q, PIK3CA:p.E545K, PIK3CA:p.H1047R, PLXNA3:p.V14fs, POSTN:p.R508C, PTPRU:p.D1434N, PYGO2:p.Q150fs, RBBP7:p.E274K, SFPQ:p.R611Q, SGSM1:p.F1117L, SLC25A40:p.R96Q, SLC8A1:p.R431H, SLITRK3:p.S298L, SPATA22:p.S150L, SUN3:p.E128K, TGFBR1:p.S241L, TP53:p.R273H, TP53:p.R273C, TP53:p.R248W, TRPVS:p.R492H, USP40:p.S851L, VPS13C:p.D1359Y, ZBTB24:p.L607I, ZNF434:p.R306C, ZNF443:p.R301I, ZNF484:p.R138C, and ZNF770:p.S441P.

In some embodiments, the neoantigen and HLA allele are selected from the following neoantigen and HLA allele pairs: TP53 (R175H)-A*02:01, TP53 (Y220C)-A*02:01, and TP53 (R248W)-A*68:01.

In some embodiments, each subject profile further comprises tumor imaging data and/or subject medical record information. In some embodiments, the subject medical record information comprises subject diagnosis information, prognosis information, cancer staging information, and/or cancer treatment history information.

In embodiments, each subject in the plurality of subjects have a viral infection, wherein the viral infection is the same, and the peptide of interest is an epitope derived from an antigen of the virus.

In some embodiments, the expressing step c) comprises expressing the TCR library in a plurality of cells, wherein an individual cell of the plurality of cells express a candidate TCR of the plurality of candidate TCRs. In some embodiments, the assessing step d) comprises contacting the plurality of cells expressing the candidate TCRs with peptide of interest/MHC (pMHC) multimers and isolating cells that bind the pMHC multimers. In certain embodiments, the cells that bind the pMHC multimers are isolated using a cell sorting method. In some embodiments, cell sorting method is selected from magnetic-activated cell sorting (MACS), fluorescence-activated cell sorting (FACS), and buoyancy-activated cell sorting. In some embodiments, the pMHC multimers are tetramers.

In some embodiments, one or more candidate TCRs of the plurality of candidate TCRs comprises: a) a variant TCR α-chain of an enriched α chain identified in step a); and/or a variant TCR β-chain of an enriched β-chain identified in step a). In some embodiments, the variant TCR α-chain has the CDR3 sequence of an enriched TCR α-chain identified in step a). In some embodiments, the variant TCR α-chain does not have the CDR1 sequence and/or CDR2 sequence of an enriched TCR α-chain identified in step a). In some embodiments, variant β-chain has the CDR3 sequence of an enriched β-chain identified in step a). In some embodiments, the variant β-chain does not have the CDR1 sequence and/or CDR2 sequence of an enriched β-chain identified in step a). In some embodiments, the variant TCR α-chain is at least about 70%, at least about 80%, at least about 90%, at least about 95%, or at least about 98% identical to an enriched α-chain identified in step a). In some embodiments, the variant TCR β-chain is at least about 70%, at least about 80%, at least about 90%, at least about 95%, or at least about 98% identical to an enriched TCR β-chain identified in step a).

In some embodiments, one or more candidate TCRs of the plurality of candidate TCRs comprises: a) a variant TCR γ-chain of an enriched α chain identified in step a); and/or b) a variant TCR δ-chain of an enriched β-chain identified in step a).

In some embodiments, the variant TCR γ-chain has the CDR3 sequence of an enriched TCR γ-chain identified in step a). In certain embodiments, the variant TCR γ-chain does not have the CDR1 sequence and/or the CDR2 sequence of an enriched TCR γ-chain identified in step a). In some embodiments, the variant δ-chain has the CDR3 sequence of an enriched δ-chain identified in step a). In some embodiments, the variant δ-chain does not have the CDR1 sequence and/or the CDR2 sequence of an enriched δ-chain identified in step a). In some embodiments, wherein the variant TCR γ-chain is at least about 70%, at least about 80%, at least about 90%, at least about 95%, or at least about 98% identical to an enriched γ-chain identified in step a). In some embodiments, the variant TCR δ-chain is at least about 70%, at least about 80%, at least about 90%, at least about 95%, or at least about 98% identical to an enriched TCR δ-chain identified in step a).

In embodiments, the method further comprises sequencing candidate TCRs that are determined to bind to a peptide of interest in complex with the HLA. In some embodiments, the sequencing is a next generation sequencing (NGS) method.

### BRIEF DESCRIPTION OF THE DRAWINGS

Figure 1 provides a non-limiting overview of the steps for identification of candidate TCRs that bind to a neoantigen/HLA allele according to some embodiments of the subject screening method.
Figure 2 depicts the interaction of TCR with peptide antigen presented in the context of an HLA class I molecule.
Figure 3 shows the results of the identification of candidate TCRs that potentially bind to TP53 R175H/HLA-A*2.01 according to the subject in silico screening methods. After clustering, the TCR space was reduced by ~4 orders of magnitude (i.e., a factor of ∼ 10,000).
Figure 4 provides a non-limiting overview of the steps for experimentally testing TCR/neoantigen according to some embodiments of the subject screening method.

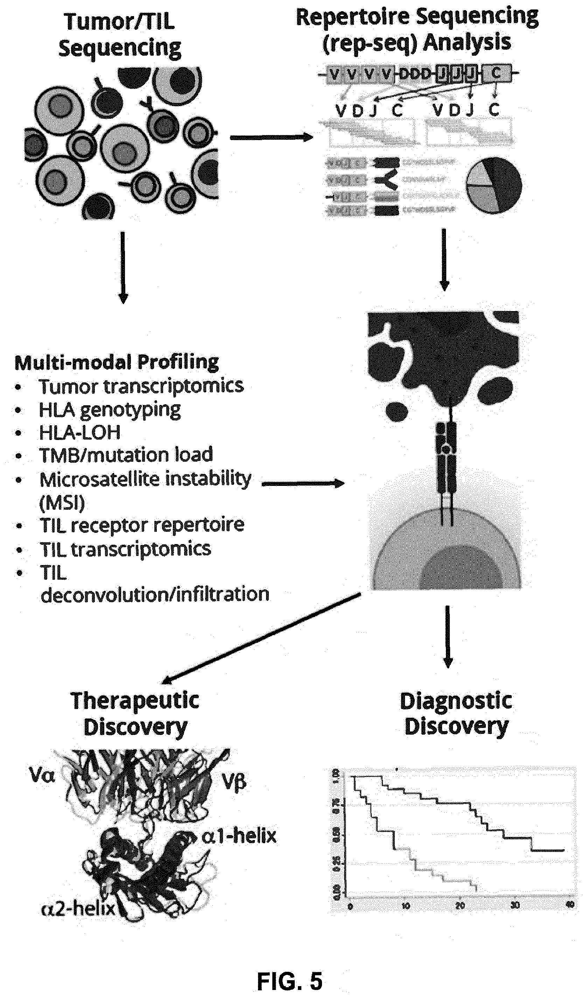

Figure 5 provides a schematic of an exemplary
   of the subject methods provided herein.
Figures 6A, 6B and 6C provide the summary of a study of the characterization of HPV associated TCRs and the associated tumor microenvironment.
Figures 7A and 7B provide a summary of a study of the identification of neoantigen specific TCRs.
Figures 8A, 8B, 8C, 8D and 8E provide the summary of a study showing that tumor immune repertoire changes over time can capture treatment associated responses.
FIG. 9 illustrates an exemplary system for candidate TCRs that are capable of binding to a peptide of interest/HLA complex, in accordance with some embodiments of the present disclosure.
FIGs. 10A, 10B, 10C, 10D, 10E, 10F, 10G, 10H, 10I, 10J, 10K provide an example flowchart depicting an example process for determining identifying TCRs capable of binding to a peptide of interest/HLA complex, in accordance with some embodiments of the present disclosure.

### DETAILED DESCRIPTION

Provided herein, inter alia, is a novel TCR screening method that finds particular use in the development of TCR-based cancer therapeutics. The subject TCR screening method, in embodiments, utilizes clustering analysis in combination with a robust subject database (e.g., a cancer patient database) to effectively identify candidate TCRs that recognize a peptide of interest (e.g., an epitope derived from a tumor-associated antigen or a neoantigen) in complex with an HLA molecule of a particular HLA allele. In a first step of embodiments, the screening method, a peptide of interest and HLA allele pair is identified. In various embodiments, a robust subject profile database is then used in combination with clustering analysis to identify TCR chain sequences (e.g., TCR α-, β-, γ-, or δ-chain sequences, or combinations thereof) that are prevalent in subjects that have the peptide of interest and HLA allele pair. In embodiments, a candidate TCR library based on the identified prevalent TCR chains is subsequently made. In embodiments, the candidate TCR library includes polynucleotides encoding the identified prevalent TCR chain sequences. In embodiments, candidate TCRs that include identified TCR chain sequences of the candidate TCR library are expressed on T cells and assessed for binding to the antigen/HLA allele complex.

Aspects of the subject TCR screening method are further discussed in detail below.

### I. Definitions

In order that the application may be more completely understood, several definitions are set forth below. Such definitions are meant to encompass grammatical equivalents.

The term "a" or "an" refers to one or more of that entity, i.e. can refer to a plural referent. As such, the terms "a" or "an," "one or more" and "at least one" are used interchangeably herein. In addition, reference to "an element" by the indefinite article "a" or "an" does not exclude the possibility that more than one of the elements is present, unless the context clearly requires that there is one and only one of the elements.

As used herein the term "antigen" is a substance that induces an immune response.

As used herein the term "neoantigen" is a tumor-specific antigen that has at least one alteration that makes it distinct from the corresponding wild-type, parental antigen, e.g., via mutation in a tumor cell or post-translational modification specific to a tumor cell. A neoantigen is present in a subject's tumor cell or tissue but not in the subject's corresponding normal cell or tissue. A neoantigen can include a polypeptide sequence or a nucleotide sequence. A mutation can include a frameshift or nonframeshift indel, missense or nonsense substitution, splice site alteration, genomic rearrangement or gene fusion, or any genomic or expression alteration giving rise to a neoORF. A mutation can also include a splice variant. Post-translational modifications specific to a tumor cell can include aberrant phosphorylation. Post-translational modifications specific to a tumor cell can also include a proteasome-generated spliced antigen. See Liepe et al., A large fraction of HLA class I ligands are proteasome-generated spliced peptides; Science. 2016 Oct. 21; 354(6310):354-358.

As used herein, the term "nucleic acid" refers to a polymeric form of nucleotides of any length, either ribonucleotides or deoxyribonucleotides, or analogs thereof. This term refers to the primary structure of the molecule, and thus includes double- and single-stranded DNA, as well as double- and single-stranded RNA. It also includes modified nucleic acids such as methylated and/or capped nucleic acids, nucleic acids containing modified bases, backbone modifications, and the like. The terms "nucleic acid" and "nucleotide sequence" are used interchangeably.

The term percent "identity," in the context of two or more nucleic acid or polypeptide sequences, refers to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared. For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

It is noted that the claims may be drafted to exclude any optional element. As such, this statement is intended to serve as an antecedent basis for use of such exclusive terminology as "solely," "only," and the like in connection with the recitation of claim elements or use of a "negative" limitation. As will be apparent to those of skill in the art upon reading this disclosure, each of the individual embodiments described and illustrated herein has discrete components and features readily separated from or combined with the features of any of the other several embodiments without departing from the scope or spirit of the invention. Any recited method may be carried out in the order of events recited or in any other order that is logically possible. Although any methods and materials similar or equivalent to those described herein may also be used in the practice or testing of the invention, representative illustrative methods and materials are now described.

Ranges provided herein are understood to be shorthand for all of the values within the range. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50, as well as all intervening decimal values between the aforementioned integers such as, for example, 1.1, 1.2, 1.3, 1.4, 1.5, 1.6, 1.7, 1.8, and 1.9. With respect to sub-ranges, "nested sub-ranges" that extend from either end point of the range are specifically contemplated. For example, a nested sub-range of an exemplary range of 1 to 50 may comprise 1 to 10, 1 to 20, 1 to 30, and 1 to 40 in one direction, or 50 to 40, 50 to 30, 50 to 20, and 50 to 10 in the other direction.

Unless specifically stated or obvious from context, as used herein, the term "about" is understood as within a range of normal tolerance in the art, for example within 2 standard deviations of the mean. About can be understood as within 50%, 45%, 40%, 35%, 30%, 25%, 20%, 15%, 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

Before the invention is further described, it is to be understood that this invention is not limited to particular embodiments described, as such may, of course, vary. It is also to be understood that the terminology used herein is for describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are encompassed within the invention, subject to any specifically excluded limit in the stated range. Where the stated range includes one or both of the limits, ranges excluding either or both of those included limits are also included in the invention. Certain ranges are presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number, which, in the context presented, provides the substantial equivalent of the specifically recited number.

All publications, patents, and patent applications cited in this specification are incorporated herein by reference to the same extent as if each individual publication, patent, or patent application were specifically and individually indicated to be incorporated by reference. Furthermore, each cited publication, patent, or patent application is incorporated herein by reference to disclose and describe the subject matter in connection with which the publications are cited. The citation of any publication is for its disclosure prior to the filing date and should not be construed as an admission that the invention described herein is not entitled to antedate such publication by virtue of prior invention. Further, the dates of publication provided might be different from the actual publication dates, which may need to be independently confirmed.

### II. Identification of Peptide of Interest/HLA allele

In embodiments of the subject TCR screening methods, a peptide of interest/HLA allele combination is identified. In some embodiments, the peptide of interest is an epitope derived from a tumor associated antigen that is expressed by unmutated genes at greater levels in tumor cells as compared to non-tumor cells. In some embodiments, the peptide of interest is an epitope derived from a tumor-specific antigen. In exemplary embodiments, the peptide of interest is an epitope derived from a neoantigen. Neoantigens are tumor-specific and considered non-self by the host immune system. Accordingly, neoantigens are effective targets for TCR-based therapeutics. In some embodiments, the peptide is associated with a viral antigen (e.g., a viral antigen associated with a cancer) or an autoreactive T-cell.

TCR receptors recognize peptide epitopes that are displayed by either major histocompatibility complex (MHC) class I or class II molecules. MHC class I molecules are found on the cell surface of all nucleated cells in the bodies of vertebrates. MHC class I molecules are heterodimers that consist of two polypeptide chains, α and β2-microglobulin (B2M). MHC class I molecules function to display peptide fragments of antigen (i.e., epitopes) to cytotoxic T cells, resulting in an immediate response from the immune system against a particular epitope displayed within the peptide-binding groove of an MHC-I molecule. In humans, MHC class I molecules include the highly polymorphic human leukocyte antigens HLA-A, HLA-B, HLA-C and the less polymorphic HLA-E, HLA-F, HLA-G, HLA-K and HLA-L. Each human leukocyte antigen (e.g., HLA-A) includes multiple alleles. For example, HLA-A includes over 2,430 non-redundant known alleles. MHC class II molecules are normally found only on antigen-presenting cells such as dendritic cells, mononuclear phagocytes, some endothelial cells, thymic epithelial cells, and B cells. MHC class I molecules are heterodimers that consist of two homogenous polypeptides, an α and β chain. In humans, the MHC class II proteins include HLA-DP, HLA-DM, HLA-DOA, HLA-DOB, HLA-DQ, and HLA-DR.

Exemplary antigen/HLA allele pairs used in the subject TCR screening methods can be identified by any suitable method in the art. Exemplary antigen/HLA allele pairs useful in the subject methods include, but are not limited to those depicted in Table 1.

**Table 1: Exemplary antigen HLA pairs**

| Antigen | HLA Allele |
|---|---|
| | |
| BRAF:p.V600E | A*02 |
| KRAS:p.G12D | A*03 |
| KRAS:p.G12V | A*03:01 |
| TP53:p.R175H | A*02:01 |
| TP53:p.Y220C | A*02:01 |
| TP53:p.R248W | A*68:01 |
| KRAS:p.G12D | A*11:01 |
| KRAS:p.G12V | B*35 |
| KRAS:p.Q61R | A*01:01 |
| KRAS:p.G12V | A*11:01 |
| BRAF:p.V600E | B*27:05 |
| KRAS:p.G12D | C*08:02 |
| HPV viral antigens | A*02:01 |
| KRAS+ | C*03:04 |
| KRAS+ | A*11:01 |

Additional antigens that can be used with the subject screening methods include, but are not limited to: KRAS:p.G12D, BRAF:p.V600E, KRAS:p.G12V, ACVR2A:p.K435fs, GRB14:p.KKK295del, SEC63:p.L532fs, TGFBR2:p.E125fs, ATR:p.K771fs, ICAl:p.N204fs, KRAS:p.G12C, TP53:p.R175H, ABCA8:p.R842Q, ACTL7B:p.R354H, ACVR2A:p.K435fs, AIM2:p.K340fs, ALG2:p.S302Y, ANKIB 1 :p.K144fs, ARSG:p.V131I, ATP10D:p.R311H, AXIN2:p.W663fs, C5orf30:p.D4N, CACNG3:p.V134I, CASP5:p.K78fs, CC2D2A:p.R1284C, CDH10:p.E349K, DNMT1:p.E432K, DOCK2:p.G170R, DOCKS:p.E177K, EGR2:p.R390H, ERBB3:p.V104M, FAM135B:p.R884H, FBXW7:p.R505C, FBXW7:p.R465H, FHDC1:p.R254W, FOXL1:p.N89K, HCN4:p.R525H, HLA-DMA:p.E84K, HTR3B:p.R236C, ITGA4:p.T673M, KIF18A:p.R17C, KIF20B:p.E991K, KLHL5:p.R326C, KRAS:p.A146T, KRAS:p.G13D, LPHN3:p.R1183Q, MAP2K4:p.R287H, MAPK8IP1:p.L217fs, MFSD5:p.R280Q, MUC16:p.R8606H, MYO6:p.D1180N, NAA25:p.S807Y, NBPF14:p.V44L, NRAS:p.Q61K, NRAS:p.G13R, PAX3:p.T424M, PGAM1:p.R240H, PHF3:p.R1410I, PIK3CA:p.R88Q, PIK3CA:p.E545K, PIK3CA:p.H1047R, PLXNA3:p.V14fs, POSTN:p.R508C, PTPRU:p.D1434N, PYGO2:p.Q150fs, RBBP7:p.E274K, SFPQ:p.R611Q, SGSM1:p.F1117L, SLC25A40:p.R96Q, SLC8A1:p.R431H, SLITRK3:p.S298L, SPATA22:p.S150L, SUN3:p.E128K, TGFBR1:p.S241L, TP53:p.R273H, TP53:p.R273C, TP53:p.R248W, TRPVS:p.R492H, USP40:p.S851L, VPS13C:p.D1359Y, ZBTB24:p.L607I, ZNF434:p.R306C, ZNF443:p.R301I, ZNF484:p.R138C, and ZNF770:p.S441P.

In some embodiments, the candidate peptide of interest is an epitope derived from a neoantigen (i.e., a neoepitope). Neoepitopes useful for practice with the subject TCR screening methods can also be identified through immunogenomic or immunopeptidomic approaches. In some embodiments of the immunogenomic method, tumor and matched germinal tissues are subjected to exome and tumor RNA-sequencing to detect somatic mutations in genes that alter the amino acid sequence of the expressed gene. Overlapping mutation peptide sequences (e.g., missense or indel mutations) are analyzed to predict affinity to MHC I or MHC II alleles. In some embodiments of the immunopeptidomic method, tumor tissues are lysed, and peptide/MHC complexes are purified by immunoprecipitation using anti-MHC antibodies. Binding peptides are eluted and separated by size. In some embodiments, mass spectrometry is then performed to determine molecular weight and identify corresponding mutated peptides. Using candidate neoepitope peptides, T cell responses are investigated by evaluating immunogenicity (e.g., cytokine production, activation marker expression, and tetramer staining) using standard techniques. Neoepitope candidates can be further selected based on their likelihood to be processed and presented on the cell surface HLA molecules using in silico prediction algorithms. Methods of identifying neoepitopes and MHC binding pairs are further disclosed for example, in Garcia-Garijo et al., Front Immuno. 10:1392 (2019); Hutchinson and Pritchard, Mamm Genome 29(11):714-730 (2018); and US Patent Nos. 11,183,286B2 and 11,264,117B2.

In some embodiments, a candidate peptide of interest/HLA allele pair (e.g., tumor neoantigen/HLA allele pair) of use in the subject screening methods is selected from known peptide of interest/HLA allele pairs, for example, peptide of interest/HLA allele pairs described in the literature. In some embodiments, candidate peptide of interest/HLA allele pairs are identified using a cancer patient database. In exemplary embodiments, the cancer patient database includes related patient HLA type, tumor mRNA expression, germline DNA and tumor DNA data, or a subset of such data for each patient in the database. In some embodiments, the database includes data for at least 50, at least 100, at least 200, at least 300, at least 400, at least 500, at least 600, at least 700, at least 800, at least 900, at least 1,000, at least 2,500, at least 5,000, at least 7,500, or at least 10,000 patients. Candidate peptide of interest/HLA allele pairs are identified from such a patient database using any suitable known in the art. In some embodiments, candidate neoepitope/HLA allele pairs are identified by identifying HLA allele/mutation pairings that are statistically enriched in a cancer patient database as compared to the frequency of the HLA allele/mutation pairing in a control population. Candidate HLA alleles that are likely to bind a particular peptide of interest can be further tested and validated using standard peptide MHC binding assays. See, e.g., Sette et al., Molecular Immunology 31(11):813-822 (1994), Rothbard and Gefter, Annual Review of Immunology 9:527-565 (1991).

Neoepitopes can be identified using samples and patient databases of patients with a particular type of cancer. In some embodiments, the cancer is selected from urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer.

### III. Identification of Candidate TCRs

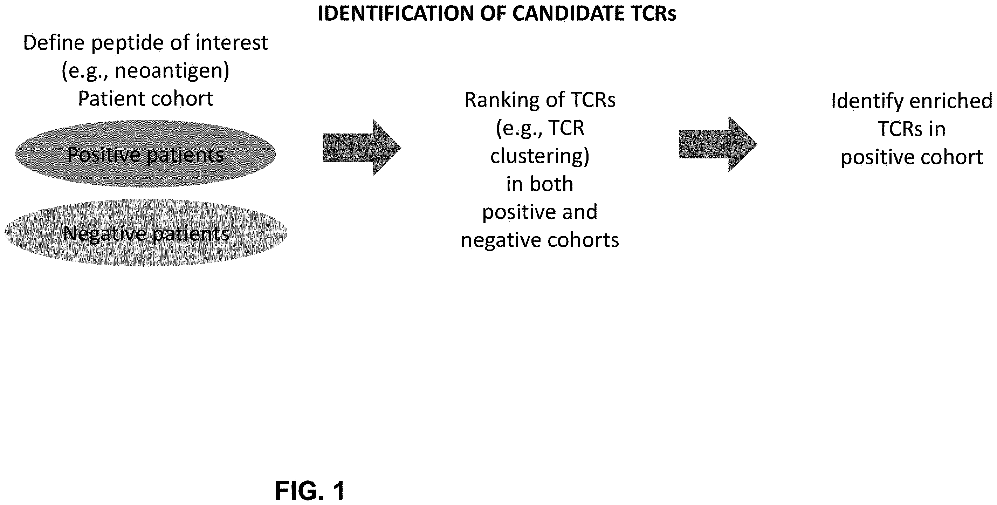

Once a particular peptide of interest/HLA allele pair is selected for use in the subject TCR screening methods, candidate T cell receptors that bind the peptide of interest/HLA allele are determined (see, e.g., Figure 1). Current screening methods generally depend on a mass screening approach, wherein a large recombinant TCR library is constructed (10¹⁵ - 10¹⁶ potential TCR combinations) and used to identify individual TCRs that bind to an peptide of interest. Such screens are labor-intensive, inefficient and costly. The TCR screening methods provided herein utilize a robust subject profile database in combination with analysis techniques (e.g, cluster analysis techniques) to advantageously preselect for candidate TCRs that are likely to bind the MHC displayed peptide of interest and reduce the size of the recombinant TCR library for experimental testing, thereby allowing for efficient and cost-saving TCR screening.

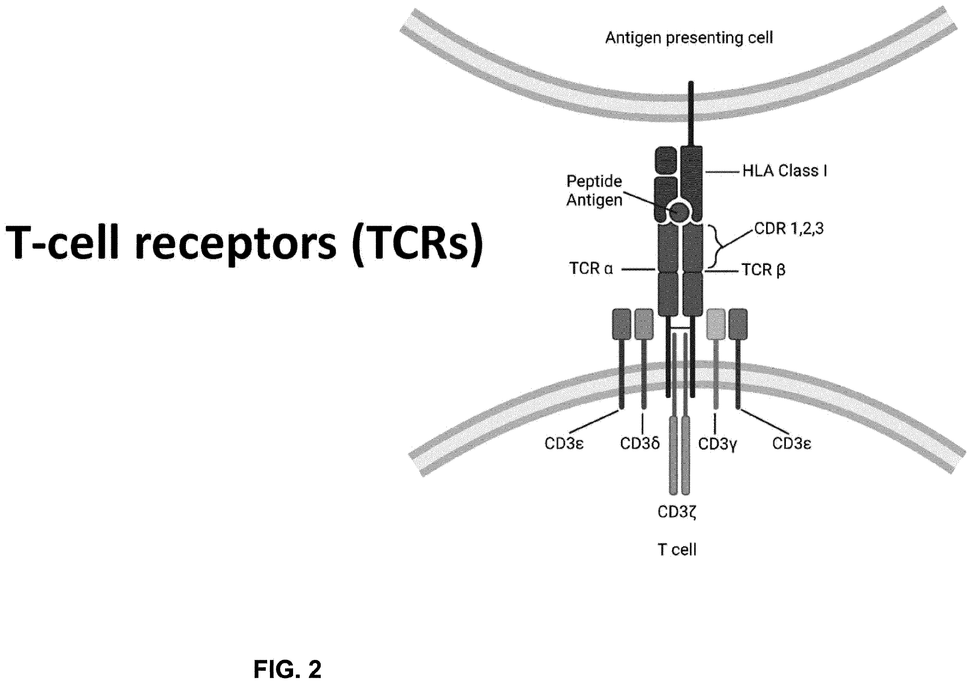

T cell receptors expressed on T cells recognize antigen peptides bound to MHC molecules (see, e.g., Figure 2). TCRs are composed of two different protein chains. In humans, the majority of TCRs consist of an alpha- (α) and a beta- (β) chain, and a minority of TCRs include a gamma- (γ) and delta- (δ) chain. Each of the TCR chains is composed of an extracellular variable domains and a constant region. The constant region further includes a transmembrane region and a cytoplasmic tail. The variable domains are involved in binding to the epitope/MHC (pMHC) complex. Upon TCR engagement with the pMHC, the T lymphocyte is activated through signal transduction.

The TCR variable domain of TCR chains each include three complementarity-determining regions (CDR1-3). CDR3 is believed to be the main CDR responsible for peptide/MHC complex recognition. TCR diversity is generated mainly from genetic recombination of the DNA-encoded segments by somatic V(D)J recombination. The TCR α- and γ-chains are generated by VJ recombination, whereas the β- and δ-chains are generated by VDJ recombination. The recombination of these specific regions (V and J for the TCR α- and γ-chain; V, D, and J for the β- and δ-chain) establishes the CDR3 region that is important for pMHC recognition.

In some embodiments, the subject profile database used for candidate TCR identification includes a plurality of subject profiles of cancer patients that each have the same cancer. In some embodiments, the subject profile database used for candidate TCR identification includes a plurality of subject profiles of patients that each have the same infectious disease (e.g., a viral, bacterial, or microbial infection). In other embodiments, the subject profile database used for candidate TCR identification includes a plurality of subject profiles of patients that each have the same autoimmune disorder, allergy, organ rejection or Graft Versus Host Disease (GHVD). In some embodiments, the methods described herein are used to identify TCRs that recognize peptide/MHC complexes wherein the peptide is an epitope derived from an antigen associated with an autoreactive T-cell in autoimmune disorder, organ rejection or GHVD.

In some embodiments, the subject profile database includes a plurality of subject profiles of cancer patients that each have the same cancer (i.e., a cancer patient profile database). In some embodiments, the cancer patient profile database includes a plurality of cancer patient profiles, wherein each patient profile includes a plurality of sequence reads. Exemplary sequence reads include but are not limited to, tumor mRNA expression, germline DNA and/or tumor DNA data, or a subset of such data for each subject in the database. In exemplary embodiments, the sequence reads include amino acid sequences derived from mRNA sequences. The mRNA expression, germline DNA, and amino acid data includes TCR data useful for identifying TCR sequences and HLA data useful for identifying HLA alleles carried and/or expressed by each subject in the database. Useful techniques for producing TCR profiles are described, for example, in US Patent No. 11,414,700, which is incorporated by reference herein and, particularly, for its disclosures relating to techniques for producing TCR profiles. Tumor DNA and mRNA expression data are useful for determining the presence of a particular peptides of interest (e.g, a neoantigen) carried by the patient. In some embodiments, the patient database includes data relating to at least about 1 × 10², about 1 × 10³, about 1 × 10⁴, about 1 × 10⁵, about 1 × 10⁶, about 1 × 10⁷, about 1 × 10⁸, about 1 × 10⁹, about 1 × 10¹⁰, about 1 × 10¹¹, or about 1 × 10¹² TCR sequences. In some embodiments, the database includes data for at least 50, at least 100, at least 200, at least 300, at least 400, at least 500, at least 600, at least 700, at least 800, at least 900, at least 1,000, at least 2,500, at least 5,000, at least 7,500, or at least 10,000 patients.

Each patient profile in the cancer patient profile database may further include additional data that may be useful in determining if a patient is more likely to have a TCR that recognizes and responds to a particular peptide of interest/MHC pair. In some embodiments, the additional data is used to pre-select for particular types of patients prior to identification of the candidate TCRs. In some embodiments, the additional data above is used to select for particular types of patients after identification of the candidate TCRs. In some embodiments, candidate TCRs are selected based on one or more of the additional data. In some embodiments, the additional data comprises information relating to patient cancer type, imaging data (e.g., MRI, CT, and histopathology imaging), patient diagnosis, staging, treatment histories, cancer stage, HLA loss of heterozygosity (LOH) status, family history, and/or biomarkers that can be used as predictors for likelihood to respond to particular treatments. In some embodiments, the cancer patient profile database includes patient profiles of cancer patients who have undergone one or more treatments for a cancer. In some embodiments, the treatment is selected from surgery, chemotherapy, immunotherapy, bone marrow transplant, immunotherapy, hormone therapy, targeted drug therapy, cryoablation, radiofrequency ablation, and combinations thereof. In some embodiments, the immunotherapy is selected from an antibody therapy, a cytokine therapy, an oncolytic virus therapy, an adoptive cell transplant therapy, a cancer vaccine or combinations thereof. In some embodiments, the antibody therapy is a checkpoint inhibitor therapy. In some embodiments, the checkpoint inhibitor therapy is an inhibitor of one of the following checkpoint targets: PD-1, PD-L1, PD-L2, CTLA-4, LAG3, TIM-3, B7H3, B7H4, A2aR, CD73, NIKG2A, PVRIG/PVRL2, CEACAM1, CECAM 5/6, FAK, CCL2/CCR2, LIF, CD47/SIRPα, CSF-1, IL-1, IL-1R3, IL-8, SEMA4D, Ang-2, CLEVER-1, Axl, and phosphatidylserine.

In some embodiments, the cancer patient profile database includes patient profiles of cancer patients selected from the following cancers: urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer.

In some embodiments, the subject profiles include data relating to biomarkers useful for predicting response to immune checkpoint inhibitor treatment. Predictive biomarker data include, but are not limited to data relating to biomarker expression (e.g., PD-1, PD-L1, PD-L2, CTLA-4, LAG3, and TIM-3 expression), tumor mutational burden (TMB), mismatch repair deficiency (dMMR), high microsatellite instability (MSI-H), and DNA damage repair (DDR) gene mutations. In some embodiments of the subject method, patient profiles are preselected for one of the following prior to further analysis: 1) patients with a particular cancer type; 2) patients that do not exhibit an HLA loss of heterozygosity (LOH) in tumor cells; 3) patients who have undergone a previous treatment (e.g., immune checkpoint inhibitor treatment); and/or 4) patients who have a particular biomarker profile that indicates unresponsiveness or responsiveness to a particular treatment.

Using the cancer patient profile database, patient profiles are selected from those that include the HLA allele identified in the peptide of interest/HLA allele pair. These patient profiles that include the HLA identified in the peptide of interest/HLA allele pair are subsequently separated into patient profiles that include the identified peptide of interest (positive subject group) and those without the identified peptide of interest (negative subject group).

In some embodiments, clustering analysis is then performed on each of the positive and negative subject groups to organize TCR α-, β-, γ- and/or δ-chain sequences for each of the defined patient groups. In some embodiments, clustering analysis is performed on each of the positive and negative subject groups to organize the TCR α-chain sequences. In some embodiments, clustering analysis is performed on each of the positive and negative subject groups to organize the TCR β-chain sequences. In some embodiments, clustering analysis is performed on each of the positive and negative subject groups to organize the TCR α- and β-chain sequences. In some embodiments, clustering analysis is performed on each of the positive and negative subject groups to organize the TCR γ-chain sequences. In some embodiments, clustering analysis is performed on each of the positive and negative subject groups to organize the TCR δ-chain sequences. In some embodiments, clustering analysis is performed on each of the positive and negative subject groups to organize the TCR γ- and δ-chain sequences.

In some embodiments, clustering analysis is performed to organize the TCR α-, β-, γ- and/or δ-chain sequences based on sequence similarity for each of the positive and negative patient groups. In exemplary embodiments, clustering analysis is performed to organize the TCR α-, β-, γ- and/or δ-chain sequences based on probability of shared epitope binding specificity for each of the positive and negative patient groups. In various embodiments, clustering analysis is performed to organize the TCR α-, β-, γ- and/or δ-chain sequences chain sequences based on sequence similarity of the α-, β-, γ- and/or δ-chain CDR1, CDR2 and/or CDR3 sequences for each of the positive and negative patient groups. In exemplary embodiments, clustering analysis is performed to organize the TCR α-, β-, γ- and/or δ-chain sequences chain sequences based on sequence similarity of the α-, β-, γ- and/or δ-chain CDR3 sequences for each of the positive and negative patient groups.

In some embodiments, clustering analysis is performed on each of the positive and negative subject groups to organize the TCR α-chain sequences based on the sequence similarity of the TCR α-chain CDR3 sequences for each of the positive and native patient groups. In some embodiments, clustering analysis is performed on each of the positive and negative subject groups to organize the TCR β-chain sequences based on the sequence similarity of the TCR β-chain CDR3 sequences for each of the positive and native patient groups. In some embodiments, clustering analysis is performed on each of the positive and negative subject groups to organize the TCR α- and β-chain sequences based on the sequence similarity of the TCR α- and β-chain CDR3 sequences for each of the positive and native patient groups. In some embodiments, clustering analysis is performed on each of the positive and negative subject groups to organize the TCR γ-chain sequences based on the sequence similarity of the TCR γ-chain CDR3 sequences for each of the positive and native patient groups. In some embodiments, clustering analysis is performed on each of the positive and negative subject groups to organize the TCR δ-chain sequences based on the sequence similarity of the TCR δ-chain CDR3 sequences for each of the positive and native patient groups. In some embodiments, clustering analysis is performed on each of the positive and negative subject groups to organize the TCR γ- and δ-chain sequences based on the sequence similarity of the TCR γ- and δ-chain CDR3 sequences for each of the positive and native patient groups.

In some embodiments, an unsupervised clustering model is used in the subject methods. In some embodiments, a supervised clustering model is used. Clustering algorithms suitable for use with the subject methods are described, for example, at pages 211-256 of Duda and Hart, Pattern Classification and Scene Analysis, 1973, John Wiley & Sons, Inc., New York, which is hereby incorporated by reference in its entirety. The clustering problem can be described as one of finding natural groupings in a dataset. To identify natural groupings, two issues can be addressed. First, a way to measure similarity (or dissimilarity) between two samples can be determined. This metric (e.g., similarity measure) can be used to ensure that the samples in one cluster are more like one another than they are to samples in other clusters. Second, a mechanism for partitioning the data into clusters using the similarity measure can be determined. One way to begin a clustering investigation can be to define a distance function and to compute the matrix of distances between all pairs of samples in the training set. If distance is a good measure of similarity, then the distance between reference entities in the same cluster can be significantly less than the distance between the reference entities in different clusters. However, clustering may not use a distance metric. For example, a nonmetric similarity function s(x, x') can be used to compare two vectors x and x'. s(x, x') can be a symmetric function whose value is large when x and x' are somehow "similar." Once a method for measuring "similarity" or "dissimilarity" between points in a dataset has been selected, clustering can use a criterion function that measures the clustering quality of any partition of the data. Partitions of the data set that extremize the criterion function can be used to cluster the data. Particular exemplary clustering techniques that can be used in the present disclosure can include, but are not limited to, hierarchical clustering (agglomerative clustering using a nearest-neighbor algorithm, farthest-neighbor algorithm, the average linkage algorithm, the centroid algorithm, or the sum-of-squares algorithm), k-means clustering, fuzzy k-means clustering algorithm, and Jarvis-Patrick clustering. In some embodiments, the clustering comprises unsupervised clustering (e.g., with no preconceived number of clusters and/or no predetermination of cluster assignments).

In some embodiments, a two-step clustering approach is used in which an N x M matrix of CDR3 amino acid sequence and physicochemical properties is sorted into superclusters using the Faiss library, and the resulting embeddings are sorted with KMeans. A graph network of distances is then produced from these superclusters based on Hamming distances between length sorted CDR3 sequences. Final cluster assignments are made by applying Markov Clustering (MCL) to the network graph. In some embodiments, multidimensional scaling (MDS) to produce matrix representations of TCR CDR3 sequences that approximate BLOSUM62 physicochemical properties, such that the Euclidean distance between two sequences represented with MDS is equivalent to the Smith-Waterman alignment between the BLOSUM representations of those sequences. MDS vectors are pre-sorted on length, and the resulting superclusters are then sorted into subclusters using the Faiss library before clustering on Smith-Waterman distances between kmers. In some embodiments, a combination of global and local cluster analyses is used. Global distance is defined as sequence mismatches in CDR3 sequences differing at a given position according to a BLOSUM62 substitution matrix, having shared TRBV gene usage and identical length. Local distance is computed as a statistically significant kmer frequency enrichment in residues predicted to contact peptide-MHC, compared to a sample population. In some embodiments, the clustering method incorporates CDR3 and (optionally) V gene usage information, pre-sorting CDR3 sequences according to length and imposing a gap penalty for length mismatched CDR3s related by a single insertion. Alignment scores are computed for a subset of the CDR3 sequences using a BLOSUM62 substitution matrix, and output clusters are assigned based on a threshold alignment score. In some embodiments, the clustering method makes use of a BLOSUM62 mismatch distance between CDR1, CDR2, CDR2.5 (an MHC-facing loop), and CDR3 sequences. Non CDR3 sequences are inferred from a reference database, a gap penalty is applied to account for sequence insertions/deletions, and a combined similarity score is computed that assigns greater weighting to CDR3 sequences. The resulting distance matrix is then clustered, for example using a greedy hierarchical search. In some embodiments, the clustering comprises clustering TCRs based on biochemical similarity, which may be done independently of consensus clustering.

Based on the cluster analysis results, TCR chain (α-, β-, γ- and/or δ-chain) sequences that are enriched in the positive control group and less prevalent or absent in the negative control group are selected to create a candidate TCR library for further screening. Any suitable method can be used to identify enriched TCR chain sequences. Exemplary methods include but are not limited to: Fisher's exact p test, Barnard's exact test, Barnard's exact test, Boschloo's test, and Cochran-Mantel-Haenszel test.

In some embodiments, a TCR chain (α-, β-, γ- or δ-chain) sequence is classified as enriched in the positive control group if the sequence is present in at least about 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, or 99% of the patient profiles in the positive control group. In some embodiments, a TCR α- or β-chain sequence is classified as not prevalent in the negative control group if the sequence is present in less than about 0.5%, 1%, 5%, 10%, 15%, 20%, 25%, 30%, 35%, 40%, 45% or 50% of the negative control group.

In some embodiments, TCR chain (α-, β-, γ- and/or δ-chain) CDR3 sequences that are prevalent in the positive control group and less prevalent or absent in the negative control group are selected to create a candidate TCR library that include such CDR3s for further screening. In some embodiments, a TCR chain (α-, β-, γ- and/or δ-chain) CDR3 sequence is prevalent in the positive control group if the sequence is present in at least about 0.1%, 0.5%, 1%, 5%, 10%, 15%, 20%, 25%, 30%, 35%, 40%, 45%, 50%, 55%, 60%, 65%, 70%, 75%, 80%, 85%, 90%, 95%, or 99% of the patient profiles in the positive control group. In some embodiments, a TCR chain (α-, β-, γ- and/or δ-chain) sequence is not prevalent in the negative control group if the sequence is present in less than about 0.5%, 1%, 5%, 10%, 15%, 20%, 25%, 30%, 35%, 40%, 45% or 50% of the negative control group. In some embodiments, TCR chain (α-, β-, γ- and/or δ-chain) CDR1 sequences that are prevalent in the positive control group and less prevalent or absent in the negative control group are selected to create a candidate TCR library that include such CDR1s for further screening. In some embodiments, TCR chain (α-, β-, γ- and/or δ-chain) CDR2 sequences that are prevalent in the positive control group and less prevalent or absent in the negative control group are selected to create a candidate TCR library that include such CDR2s for further screening.

In some embodiments, the identified enriched TCR chains (α-, β-, γ- and/or δ-chain) are further selected for enriched α/β TCR chain and/or enriched γ/δ TCR chain combinations that are expressed in the same subject (e.g., the same cancer subject).

In some embodiments, a computer system is used to carry out the identification of candidate T cell receptors that bind the antigen/HLA allele according to the methods described herein. In some embodiments, the computer system comprises at least one processor and a memory storing at least one program for execution by the at least one processor, the at least one program comprising instructions for identifying enriched TCR α-chains and TCR β-chains in cancer subjects that express the neoantigen and HLA allele using the cancer patient profile database described herein.

In various embodiments, the method is performed using an autoimmune disorder patient profile database comprising subject profiles of patients having an autoimmune disorder of interest. In some embodiments, a positive control patient group with an autoimmune disorder, and a negative control patient group without an autoimmune disorder are defined in order to determine which TCRs are more likely to be associated with the autoimmune disorder (for example, which TCRs are causing the disorder). In various embodiments, the method is performed using an allergy patient profile database comprising subject profiles of patients having an allergy of interest. In some embodiments, a positive control patient group with a particular allergy, and a negative control patient group without the allergy are defined in order to determine which TCRs are more likely to be associated with the allergy (for example, which TCRs are causing the allergy). In various embodiments, the method is performed using an infectious disease patient profile database comprising subject profiles of patients having an infectious disease of interest. In some embodiments, a positive control patient group with an infectious disease, and a negative control patient group without an infectious disease are established in order to determine which TCRs are more likely to be associated with the infectious disease (for example, which TCRs are causing the disorder) or to recognize the pathogen causing the infectious disease. Information obtained from such embodiments can be used for many downstream applications, for example, to develop treatments and/or vaccines for the autoimmune disorder, allergy, or infectious disease, to monitor the progression of the infectious disease, allergy, or autoimmune disorder, and predict severity of symptoms for the infectious disease, allergy, or autoimmune disorder.

### IV. Exemplary system embodiments for identifying TCRs capable of binding to a peptide of interest/HLA complex

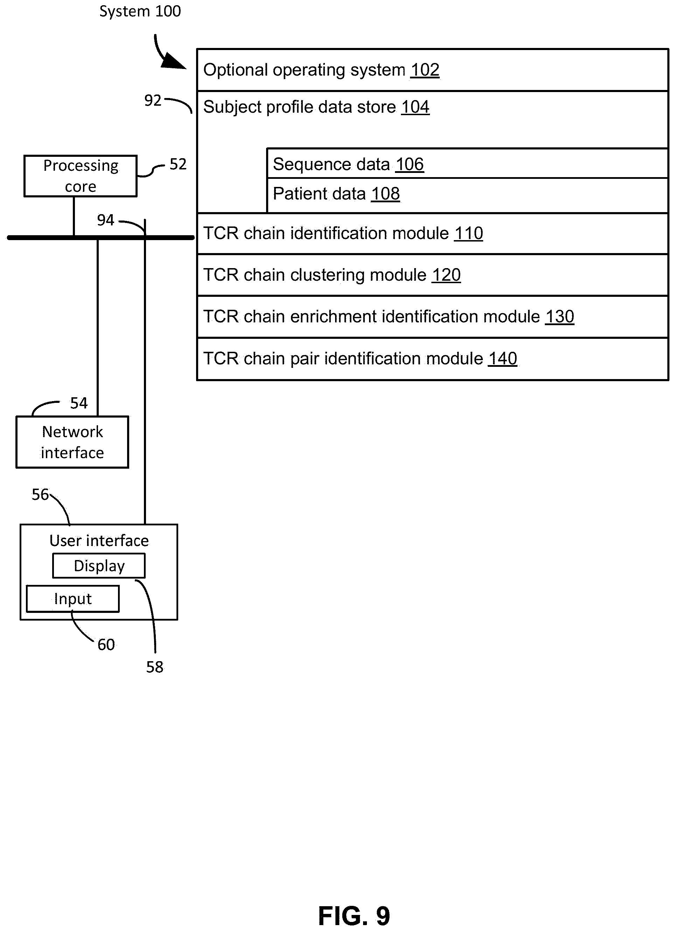

Now that an overview of some aspects of the present disclosure and some definitions used in the present disclosure have been provided, details of an exemplary system are described in conjunction with Figure 9.

Figure 9 illustrates a computer system 100 for identifying TCRs capable of binding to a peptide of interest/HLA complex. In typical embodiments, computer system 100 comprises one or more computers. For purposes of illustration in Figure 9, the computer system 100 is represented as a single computer that includes all of the functionality of the disclosed computer system 100. However, the present disclosure is not so limited. The functionality of the computer system 100 may be spread across any number of networked computers and/or reside on each of several networked computers and/or virtual machines. One of skill in the art will appreciate that a wide array of different computer topologies is possible for the computer system 100 and all such topologies are within the scope of the present disclosure.

Turning to Figure 9 with the foregoing in mind, the computer system 100 comprises one or more processing units (CPUs) 52, a network or other communications interface 54, a user interface 56 (e.g., including an optional display 58 and optional input 60 (*e.g.* keyboard or other form of input device)), a memory 92 (*e.g.,* random access memory, persistent memory, or combination thereof), and one or more communication busses 94 for interconnecting the aforementioned components. To the extent that components of memory 92 are not persistent, data in memory 92 can be seamlessly shared with non-volatile memory (not shown) or portions of memory 92 that are non-volatile / persistent using known computing techniques such as caching. Memory 92 can include mass storage that is remotely located with respect to the central processing unit(s) 52. In other words, some data stored in memory 92 may in fact be hosted on computers that are external to computer system 100 but that can be electronically accessed by the computer system 100 over an Internet, intranet, or other form of network or electronic cable using network interface 54. In some embodiments, the computer system 100 makes use of models that are run from the memory associated with one or more graphical processing units in order to improve the speed and performance of the system. In some alternative embodiments, the computer system 100 makes use of models that are run from memory 92 rather than memory associated with a graphical processing unit.

The memory 92 of the computer system 100 stores:
- an optional operating system 102 that includes procedures for handling various basic system services;
- a subject profile data store 104 comprising a plurality of subject profiles that comprise sequence data 106 for a plurality of subjects. In exemplary embodiments, the sequence data includes TCR α-chain, -β-chain, γ-chain, and/or δ-chain amino acid and/or nucleic acid sequence data. In some embodiments, the subject profile database includes additional patient data 108. Any suitable patient data that facilitates the clustering of TCR chains or identification of TCR chain pairs can be included, including any of the patient data discussed herein. In some embodiments, the patient data includes one or more of the following: patient HLA type, tumor mRNA expression, germline DNA and tumor DNA data, tumor imaging data and subject medical record information an analysis module 104 for determining whether a subject has a positive or negative molecular residual disease status for a cancer condition.
- a TCR chain identification module 110 for identifying a first plurality of sequences for a first TCR chain and a second plurality of sequences for a second TCR chain from a plurality of the sequence read profiles, wherein each respective sequence read profile in the plurality of sequence read profiles comprises corresponding sequence reads for amino acids in a biological sample from a respective subject in a plurality of subjects,

- a TCR chain clustering module 120 for clustering: 1) the first plurality of sequences into a first plurality of sequence groups based on sequence similarity between the respective sequences for the first TCR chain in the first plurality of sequences; and 2) the second plurality of sequences into a second plurality of sequence groups based on sequence similarity between the respective sequences for the second TCR chain in the second plurality of sequences;
- a TCR chain enrichment identification module 130 for identifying: 1) a first subset of the first plurality of sequence groups containing sequences that are more prevalent in a first subset of the plurality of subjects that have both the peptide of interest and the HLA than in a second subset of the plurality of subjects that do not have both the peptide of interest and the HLA; and 2) a second subset of the second plurality of sequence groups containing sequences that are more prevalent in the first subset of the plurality of subjects that have the peptide of interest and the HLA than in the second subset of the plurality of subjects that do not have the peptide of interest and the HLA; and
- a TCR chain pair identification module 140 for identifying a plurality of TCR candidate pairs, wherein each respective TCR candidate pair comprises a respective sequence for the first TCR chain present in the first subset of the first plurality of sequence groups or a variant thereof and a respective sequence for the second TCR chain present in the second subset of the second plurality of sequence groups or a variant thereof.

In some embodiments, one or more of the above identified data elements or modules of the computer system 100 are stored in one or more of the previously mentioned memory devices, and correspond to a set of instructions for performing a function described above. The above identified data, modules or programs (e.g., sets of instructions) need not be implemented as separate software programs, procedures or modules, and thus various subsets of these modules may be combined or otherwise re-arranged in various implementations. In some implementations, the memory 92 optionally stores a subset of the modules and data structures identified above. Furthermore, in some embodiments the memory 92 stores additional modules and data structures not described above.

### V. TCR Mapping to Epitopes

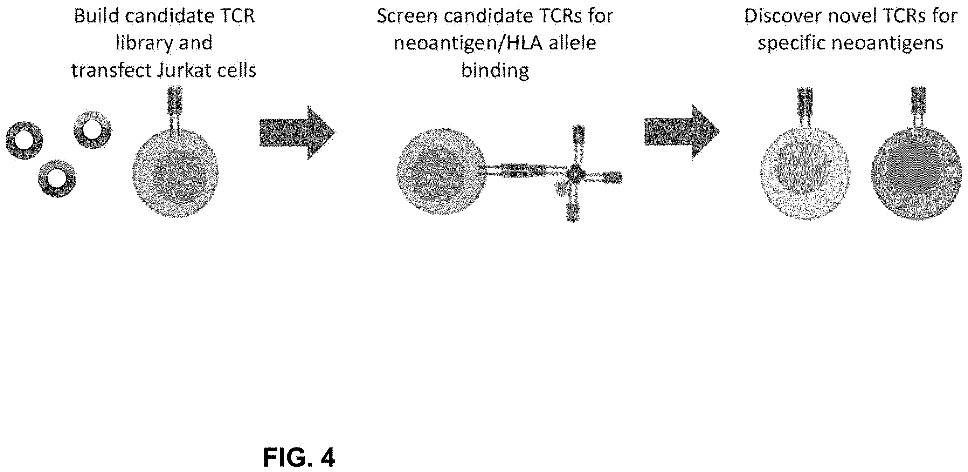

Once candidate TCR chains (α-, β-, γ- and/or δ-chains) are identified, a candidate TCR library is prepared comprising polynucleotides that encode the TCR chains or variants thereof. Candidate TCRs that include a candidate TCR chain or variants thereof are expressed on T cells, and TCRs from the candidate TCRs that bind peptide of interest/HLA allele complex are identified (see, e.g., Figure 4). In some embodiments, the candidate TCRs comprise candidate TCR α-chains, candidate TCR β-chains, or candidate TCR α- and β-chain combinations. In some embodiments, the candidate TCR chains comprise candidate TCR γ chains, candidate TCR δ-chains, or candidate TCR γ- and δ-chain combinations.

In some embodiments, a variant TCR chain (α-, β-, γ- or δ-chain) is at least about 50%, about 60%, about 65%, about 70%, about 75%, about 80%, about 85%, about 90%, about 95% or about 99% identical to the amino acid of a reference candidate TCR chain.

As TCR chain CDR3 regions function in antigen recognition, candidate TCR chains (α-, β-, γ- and/or δ-chains) variants can be designed to include the CDR3 region of a reference candidate TCR chain, and include one or more modifications outside of the CDR3 region. In some embodiments, the variant TCR chain (α-, β-, γ- or δ-chain) has the same CDR3 as a reference candidate TCR chain. In some embodiments, the variant TCR chain α-, β-, γ- or δ-chain) includes at least about 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 100, or more amino acid modifications in a non-CDR3 region as compared to a reference candidate TCR chain. In some embodiments, the variant includes the same CDR3, but a different V, D, J and/or C region as compared to a reference candidate TCR chain. In some embodiments, the variant TCR chain (α-, β-, γ- or δ-chain) includes one or more modification is in a V region or J region that is not in a CDR3 as compared to a reference candidate TCR chain (α-, β-, γ- or δ-chain). In some embodiments, the variant is a variant β- or δ- chain that includes one or more modifications in a D region as compared to a reference candidate TCR β-chain. In some embodiments, the variant TCR chain (α-, β-, γ- or δ-chain) includes one or more modifications in a C region of a reference candidate TCR chain.

In various embodiments, the CDR1, CDR2, and/or CDR3 of a known TCR chain sequence (e.g., a reference TCR α-, β-, γ- or δ-chain obtained from a TCR sequence database) is replaced with a candidate CDR1, CDR2, and/or CDR3 identified using the subject method to generate candidate TCR chains for testing. Reference TCR α-, β-, γ- or δ-chains can be obtained, for example, from the IMGT^{®}, (ImMunoGeneTics) sequence data base. In exemplary embodiments, the CDR3 region of a reference TCR chain is replaced with a candidate CDR3. In some embodiments, the CDR2 region of a reference TCR chain is replaced with a candidate CDR2. In some embodiments, the CDR1 region of a reference TCR chain is replaced with a candidate CDR1.

In some embodiments, the TCR is used to make a plurality of expression vectors, wherein the expression vectors each include polynucleotides encoding for a combination of the candidate TCR α-and β-chains, or TCR γ-and δ-chains and/or variants. The expression vectors are introduced into T cell lines (e.g. Jurkat) to produce a plurality of T cell clones that each express a TCR having a different combinations of the candidate TCR α- and β-chains, TCR γ-and δ-chains, and/or variants thereof. Each of the polynucleotides encoding the candidate TCR chains are linked to a signal protein that allows for expression of the T cell. The vector may further include one or more selectable markers for selection of T cells that include the expression vector. In various embodiments, the T cell line used for candidate TCR expression does not express endogenous TCRs. In some embodiments, the polynucleotides are introduced into the T cell line by lentiviral transduction, or the like.

Following introduction of the candidate TCR α- and β-chains and/or TCR γ- and δ-chains expression vectors into T cells, T cells that carry an expression vector can be selected for using a selectable marker included in the expression vector (e.g., a drug resistance marker). T cells that express a candidate TCR molecule can be detected by staining with a suitable marker (e.g., an antibody that binds to the constant region of a candidate TCR molecule), and isolated using FACS. In some embodiments, T cells that express a candidate TCR molecule are sequenced to determine and ensure particular combinations of candidate TCR α- and β-chains or TCR γ- and δ-chains are expressed in the T cell population. The candidate TCR expressing T cells are then assessed for binding to the peptide of interest/HLA allele complex (e.g, a neoantigen peptide/HLA allele complex).

Any suitable method may be used to assess for binding of the candidate TCRs with the peptide of interest/HLA allele complex. In some embodiments, peptide of interest /HLA allele complex binding is assessed using tetramers. In such embodiments, candidate TCR expressing T cells are contacted with multimer (e.g., tetramers) that include the peptide of interest/HLA allele complex and a detectable marker (e.g., a fluorescent marker). T cells that include express candidate TCRs that bind to the peptide of interest/HLA allele complex are subsequently isolated. In some embodiments, isolation of the T cells is performed using FACS using a fluorescent marker included in the tetramer. Candidate TCRs expressed in the isolated T cells are then sequenced to identify the candidate TCRs that are capable of binding to the peptide of interest. In some embodiments, long read sequencing is used to determine/confirm the sequence of the candidate TCRs that recognize the peptide of interest.

In some embodiments, candidate TCRs that recognize the peptide of interest (e.g, a neoepitope) undergo further validation testing to ensure that the candidate TCR function in a specific manner. In some embodiments, T cells expressing the candidate TCRs are assessed to determine if the candidate TCRs induce T cell proliferation in a specific manner via peptide of interest /HLA allele recognition. In some embodiments, the candidate TCRs are assessed for cross-reactivity to known human peptides to predict whether the candidate TCRs will react to normal and/or healthy human cells. Reactive T cells can also be assessed for specificity of peptide of interest/HLA allele expressing tumor cells using tumor organoids that express the peptide of interest /HLA allele.

### VI. First embodiment of methods for identifying TCRs capable of binding to a peptide of interest/HLA complex

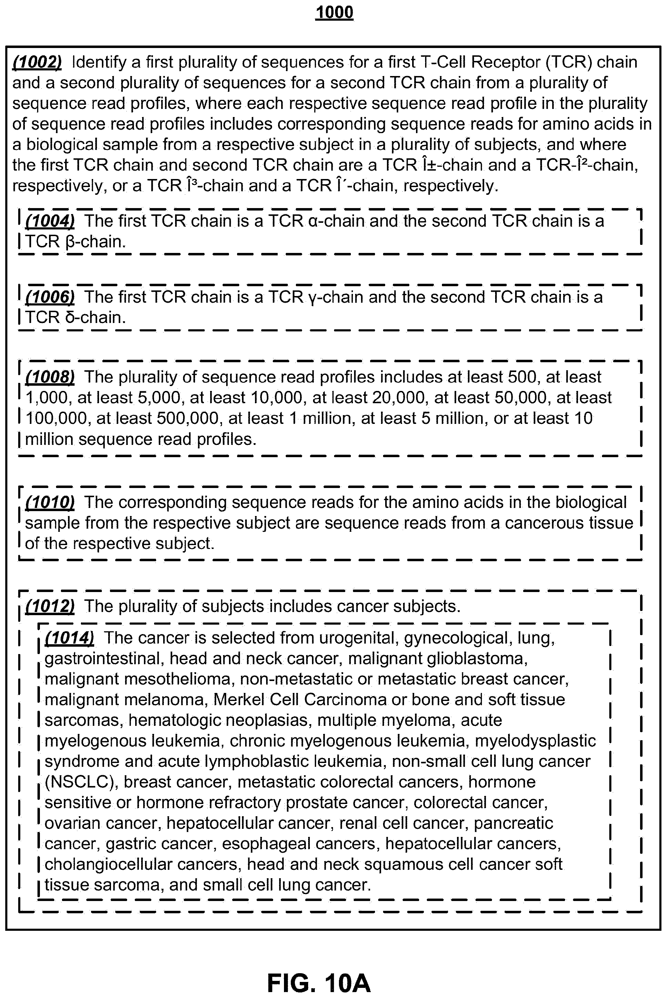

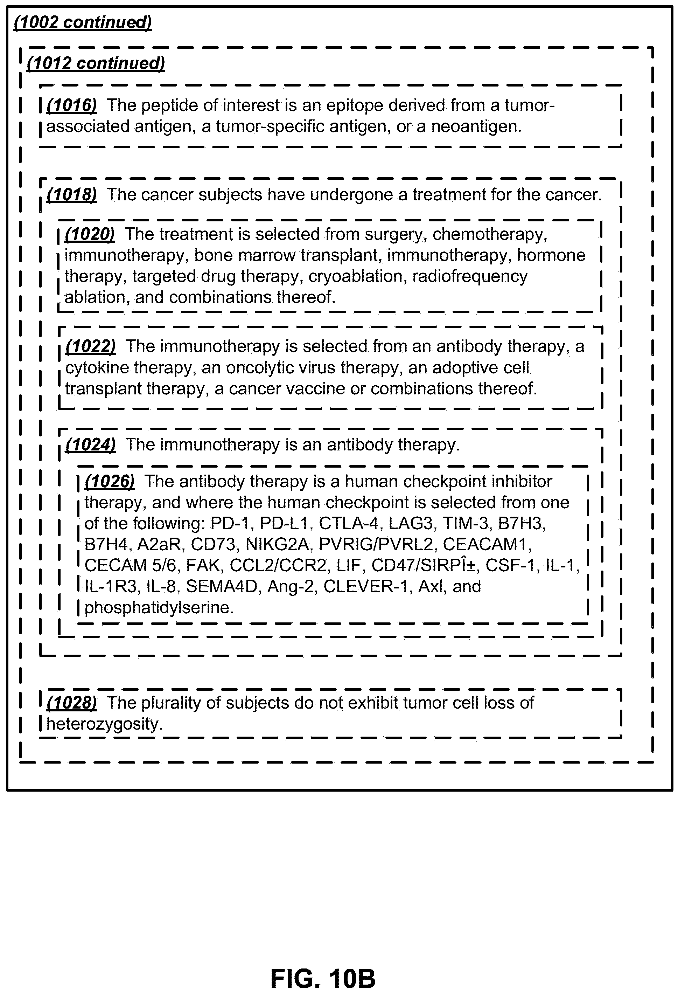

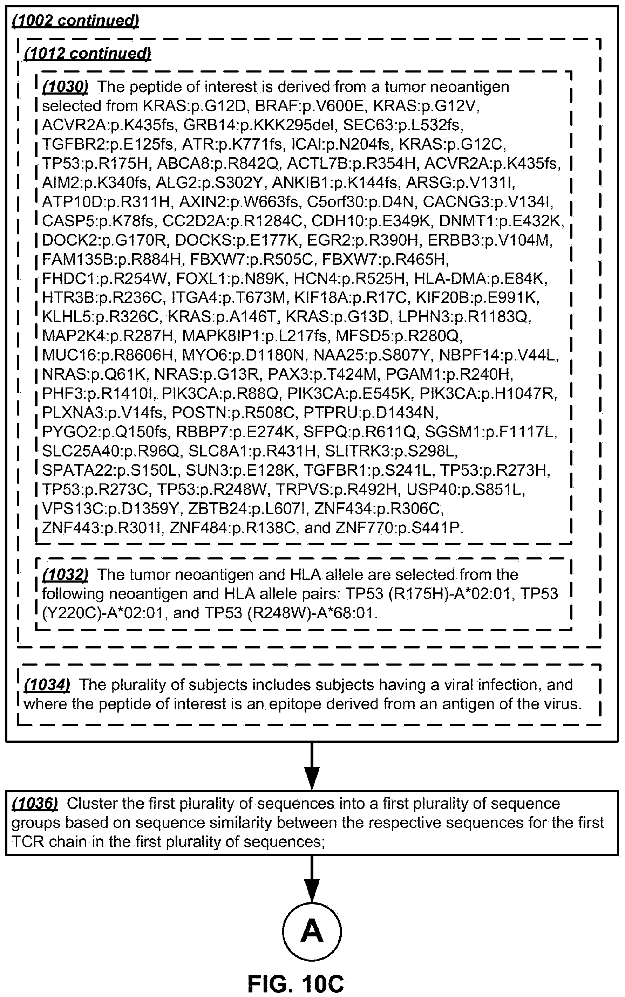

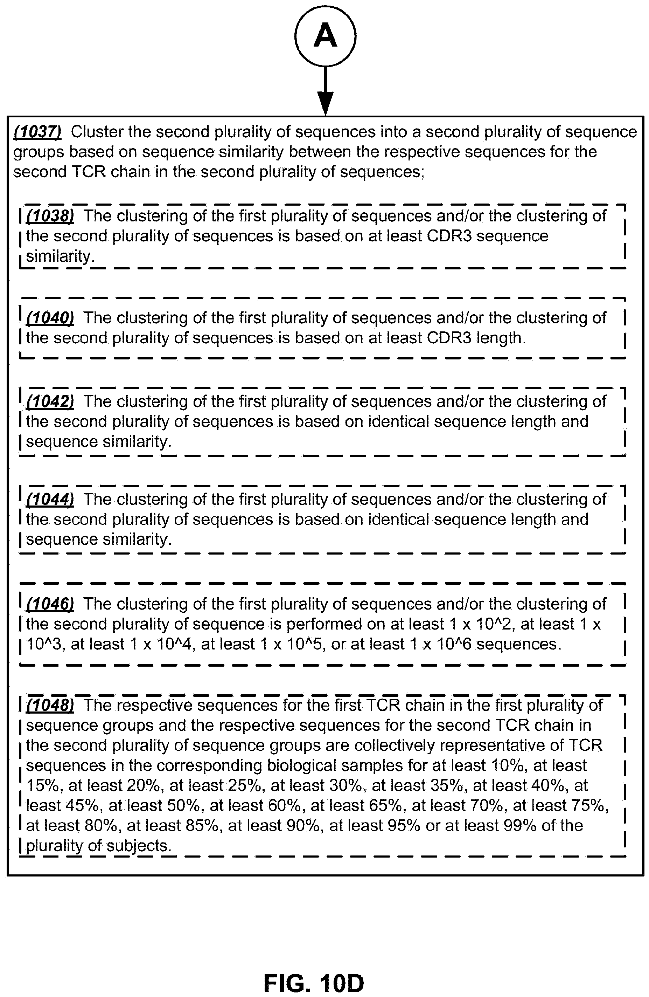

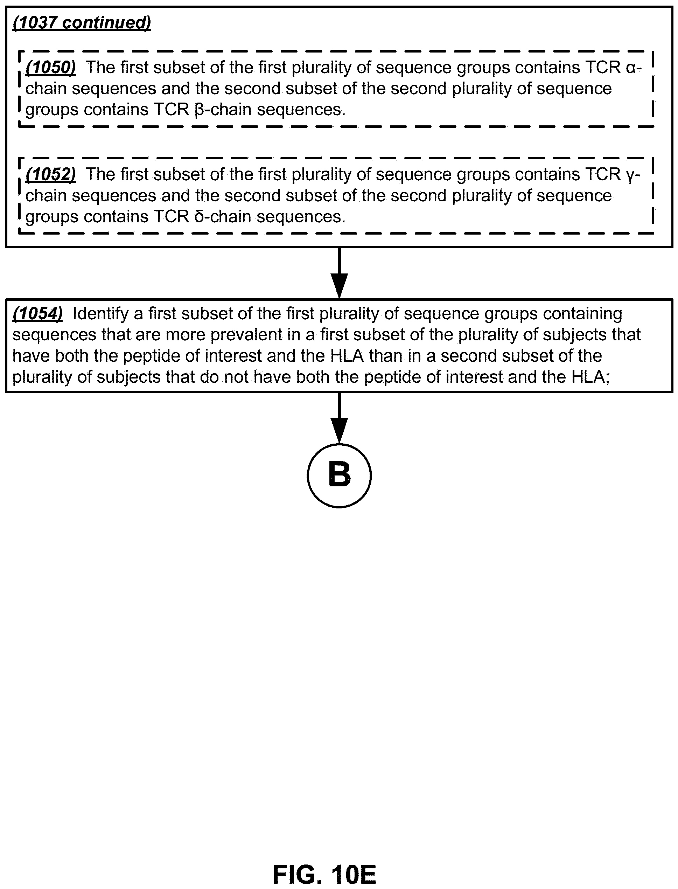

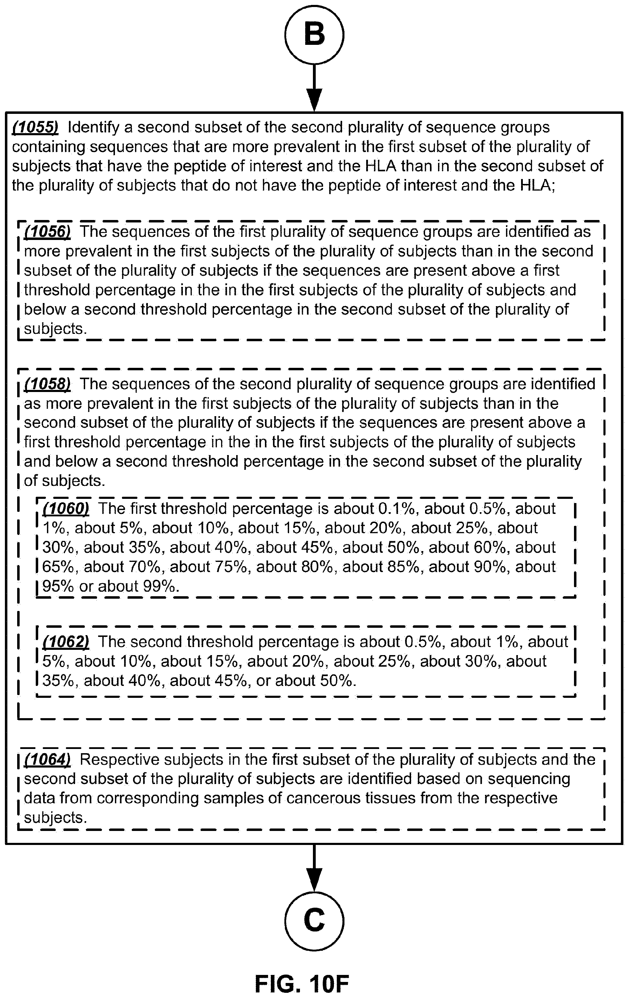

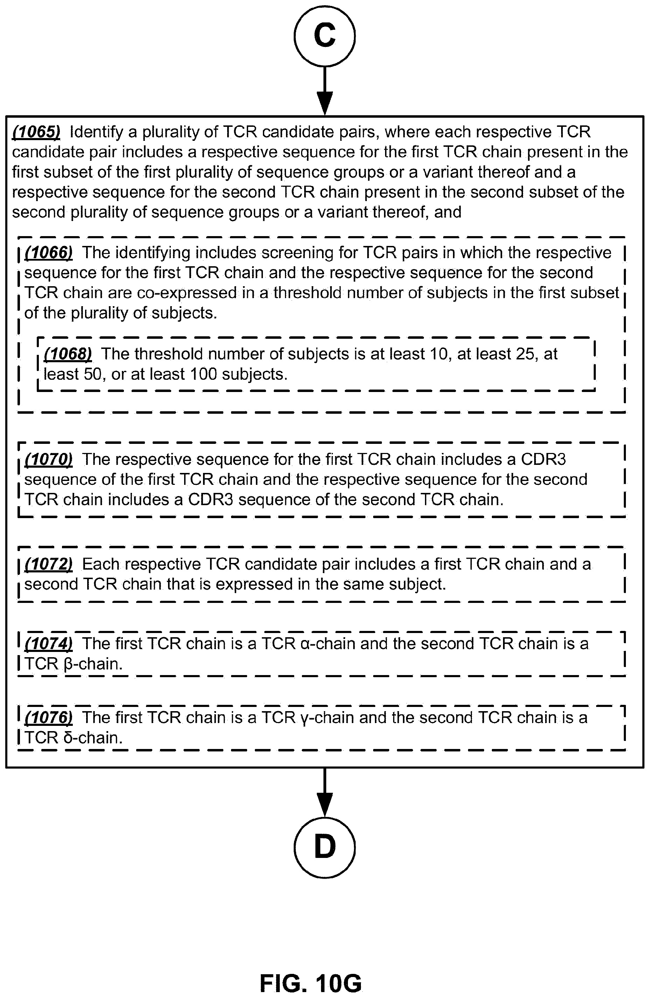

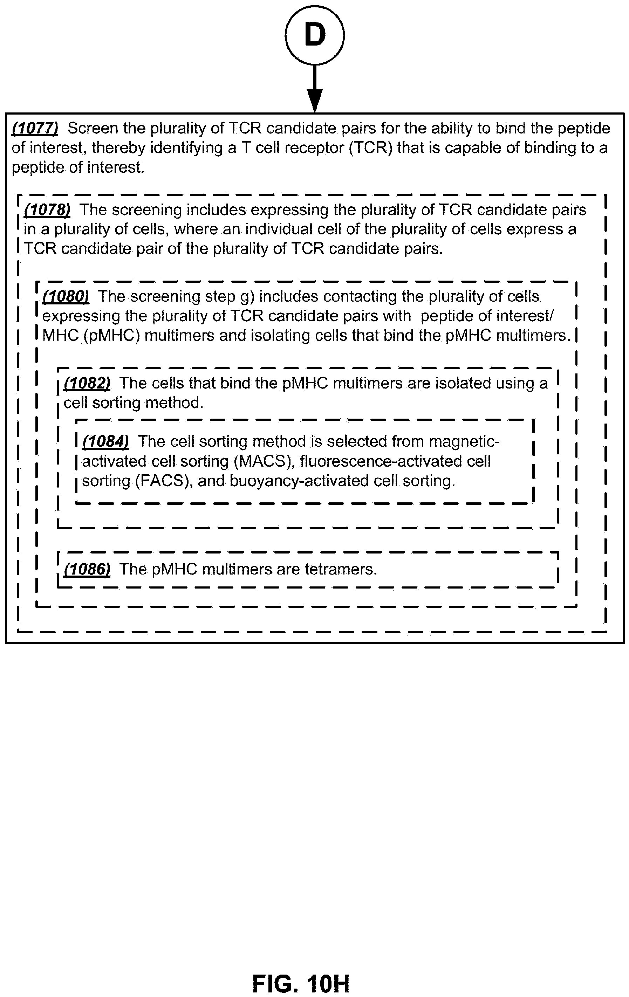

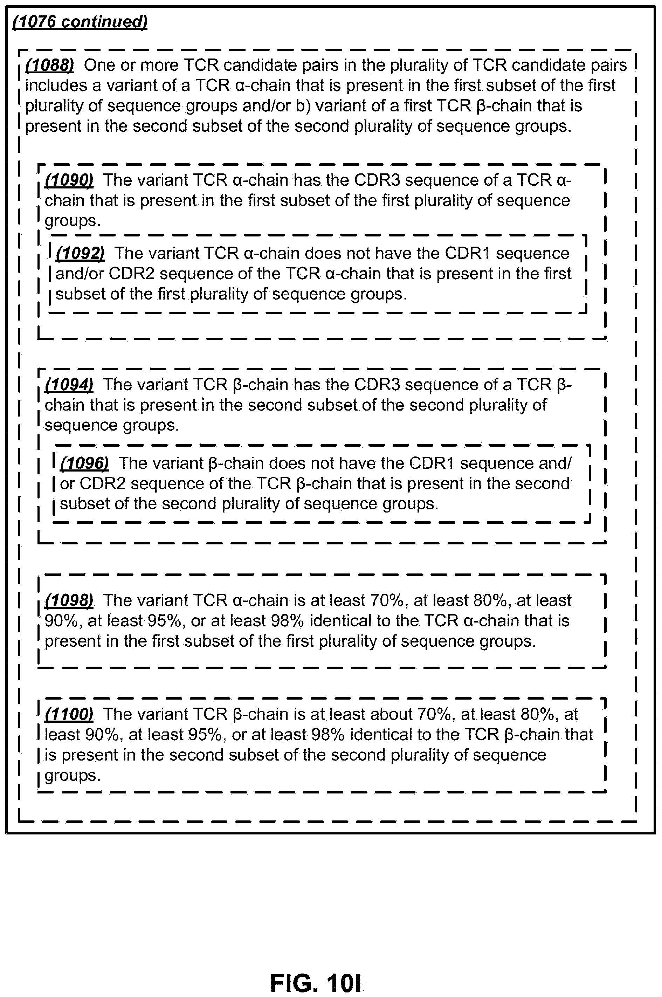

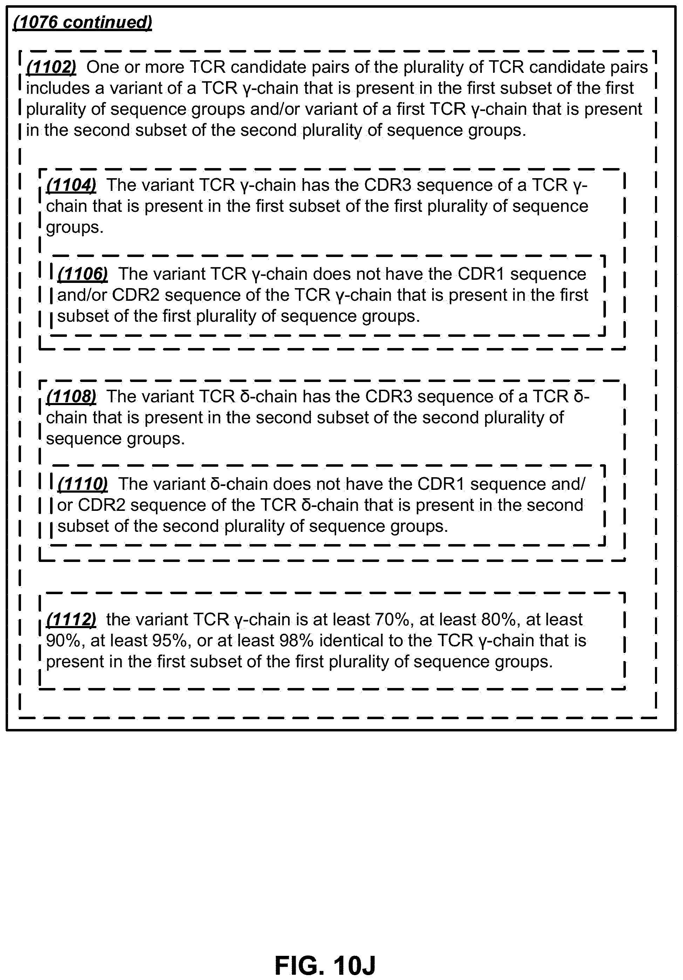

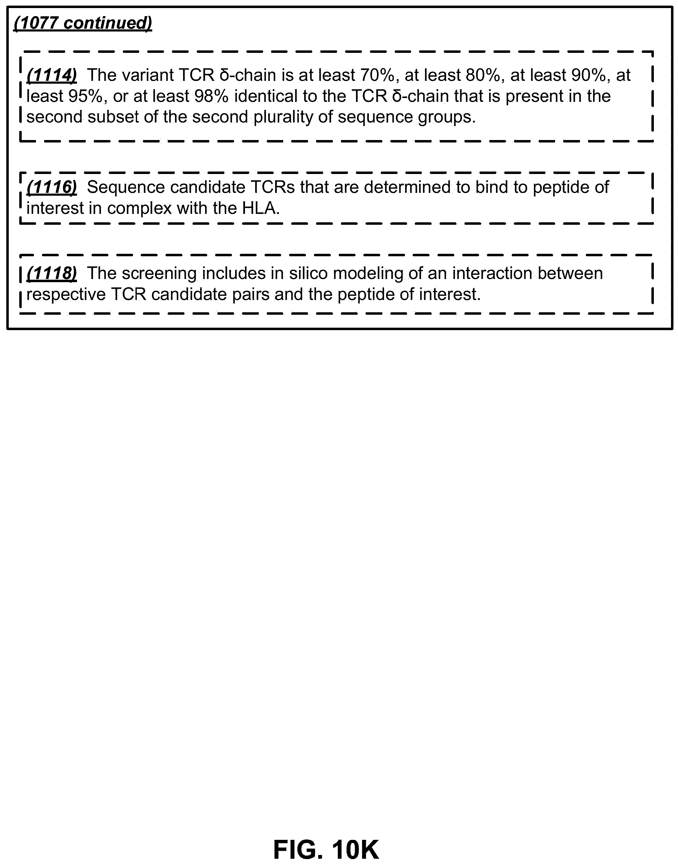

Now that a system for identifying TCRs capable of binding to a peptide of interest/HLA complex has been disclosed, methods for performing such determinations are detailed with reference to Figure 10 as discussed below.

Referring to block 1002, in some embodiments, identify a first plurality of sequences for a first T-Cell Receptor (TCR) chain and a second plurality of sequences for a second TCR chain from a plurality of sequence read profiles, where each respective sequence read profile in the plurality of sequence read profiles includes corresponding sequence reads for nucleic acids and/or amino acid sequences derived from such nucleic acid reads in a biological sample from a respective subject in a plurality of subjects, and where the first TCR chain and second TCR chain are a TCR α-chain and a TCR-β-chain, respectively, or a TCR γ-chain and a TCR δ-chain, respectively.

In some embodiments, each of the plurality of subjects has a subject profile that includes a sequence read profile and one or more additional profiles that facilitate the identification of TCRs that are capable of binding to the peptide of interest/HLA complex. In some embodiments, each subject profile further includes a TCR profile of the TCR α-chains and TCR β-chains and/or TCR γ-chains and TCR δ-chains of a subject. In some embodiments, each subject profile further includes an HLA allele profile of HLA alleles of a subject. In exemplary embodiments, the subject profile further includes a peptide of interest profile comprising information relating to the presence or amount of a peptide of interest in a subject.

In some embodiments, each subject profile further includes additional data that may be useful in determining if a patient is more likely to have a TCR that recognizes and responds to a particular peptide of interest/MHC pair. In some embodiments, the additional data is used to pre-select for particular types of patients prior to identification of the candidate TCRs. In some embodiments, the additional data above is used to select for particular types of patients after identification of the candidate TCRs. In some embodiments, candidate TCRs are selected based on one or more of the additional data. In some embodiments, the additional data comprises information relating to patient cancer type, imaging data (e.g., MRI, CT, and histopathology imaging), patient diagnosis, staging, treatment histories, cancer stage, HLA loss of heterozygosity (LOH) status, family history, and/or biomarkers that can be used as predictors for likelihood to respond to particular treatments. In some embodiments, the cancer patient profile database includes patient profiles of cancer patients who have undergone one or more treatments for a cancer. In some embodiments, the treatment is selected from surgery, chemotherapy, immunotherapy, bone marrow transplant, immunotherapy, hormone therapy, targeted drug therapy, cryoablation, radiofrequency ablation, and combinations thereof. In some embodiments, the immunotherapy is selected from an antibody therapy.

In some embodiments, the sequence read profiles are from at least 50, at least 100, at least 200, at least 300, at least 400, at least 500, at least 600, at least 700, at least 800, at least 900, at least 1,000, at least 2,500, at least 5,000, at least 7,500, or at least 10,000 subjects.

Referring to block 1004, in some embodiments, the first TCR chain is a TCR α-chain and the second TCR chain is a TCR β-chain. Referring to block 1006, in some embodiments, the first TCR chain is a TCR γ-chain and the second TCR chain is a TCR δ-chain.

Referring to block 1008, in some embodiments, the plurality of sequence read profiles includes at least 500, at least 1,000, at least 5,000, at least 10,000, at least 20,000, at least 50,000, at least 100,000, at least 500,000, at least 1 million, at least 5 million, or at least 10 million sequence read profiles.

Referring to block 1010, in some embodiments, the corresponding sequence reads for the amino acids in the biological sample from the respective subject are sequence reads for mRNA or amino acid sequence derived from such mRNA sequence reads from a cancerous tissue of the respective subject.

Referring to block 1012, in some embodiments, the plurality of subjects includes cancer subjects. Referring to block 1014, in some embodiments, the cancer is selected from urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer.

Referring to block 1016, in some embodiments, the peptide of interest is an epitope derived from a tumor-associated antigen, a tumor-specific antigen, or a neoantigen. In some embodiments, the candidate peptide of interest is an epitope derived from a neoantigen (i.e., a neoepitope). Neoepitopes useful for practice with the subject TCR screening methods can also be identified through immunogenomic or immunopeptidomic approaches. In some embodiments of the immunogenomic method, tumor and matched germinal tissues are subjected to exome and tumor RNA-sequencing to detect somatic mutations in genes that alter the amino acid sequence of the expressed gene. Overlapping mutation peptide sequences (e.g., missense or indel mutations) are analyzed to predict affinity to MHC I or MHC II alleles. In some embodiments of the immunopeptidomic method, tumor tissues are lysed, and peptide/MHC complexes are purified by immunoprecipitation using anti-MHC antibodies. Binding peptides are eluted and separated by size. In some embodiments, mass spectrometry is then performed to determine molecular weight and identify corresponding mutated peptides. Using candidate neoepitope peptides, T cell responses are investigated by evaluating immunogenicity (e.g., cytokine production, activation marker expression, and tetramer staining) using standard techniques. Neoepitope candidates can be further selected based on their likelihood to be processed and presented on the cell surface HLA molecules using in silico prediction algorithms. Methods of identifying neoepitopes and MHC binding pairs are further disclosed for example, in Garcia-Garijo et al., Front Immuno. 10:1392 (2019); Hutchinson and Pritchard, Mamm Genome 29(11):714-730 (2018); and US Patent Nos. 11,183,286B2 and 11,264,117B2. In some embodiments, neoantigens are identified using samples and patient databases of patients with a particular type of cancer.

Referring to block 1018, in some embodiments, the cancer subjects have undergone a treatment for the cancer. Referring to block 1020, in some embodiments, the treatment is selected from surgery, chemotherapy, immunotherapy, bone marrow transplant, immunotherapy, hormone therapy, targeted drug therapy, cryoablation, radiofrequency ablation, and combinations thereof. Referring to block 1022, in some embodiments, the immunotherapy is selected from an antibody therapy, a cytokine therapy, an oncolytic virus therapy, an adoptive cell transplant therapy, a cancer vaccine or combinations thereof. Referring to block 1024, in some embodiments, the immunotherapy is an antibody therapy. Referring to block 1026, in some embodiments, the antibody therapy is a human checkpoint inhibitor therapy, and where the human checkpoint is selected from one of the following: PD-1, PD-L1, CTLA-4, LAG3, TIM-3, B7H3, B7H4, A2aR, CD73, NIKG2A, PVRIG/PVRL2, CEACAM1, CECAM 5/6, FAK, CCL2/CCR2, LIF, CD47/SIRPα, CSF-1, IL-1, IL-1R3, IL-8, SEMA4D, Ang-2, CLEVER-1, Axl, and phosphatidylserine.

Referring to block 1028, in some embodiments, the plurality of subjects do not exhibit tumor cell loss of heterozygosity.

Referring to block 1030, in some embodiments, the peptide of interest is derived from a tumor neoantigen selected from KRAS:p.G12D, BRAF:p.V600E, KRAS:p.G12V, ACVR2A:p.K435fs, GRB14:p.KKK295del, SEC63:p.L532fs, TGFBR2:p.E125fs, ATR:p.K771fs, ICAl:p.N204fs, KRAS:p.G12C, TP53:p.R175H, ABCA8:p.R842Q, ACTL7B:p.R354H, ACVR2A:p.K435fs, AIM2:p.K340fs, ALG2:p.S302Y, ANKIB1:p.K144fs, ARSG:p.V131I, ATP10D:p.R311H, AXIN2:p.W663fs, C5orf30:p.D4N, CACNG3:p.V134I, CASP5:p.K78fs, CC2D2A:p.R1284C, CDH10:p.E349K, DNMT1:p.E432K, DOCK2:p.G170R, DOCKS:p.E177K, EGR2:p.R390H, ERBB3:p.V104M, FAM135B:p.R884H, FBXW7:p.R505C, FBXW7:p.R465H, FHDC1:p.R254W, FOXL1:p.N89K, HCN4:p.R525H, HLA-DMA:p.E84K, HTR3B:p.R236C, ITGA4:p.T673M, KIF18A:p.R17C, KIF20B:p.E991K, KLHL5:p.R326C, KRAS:p.A146T, KRAS:p.G13D, LPHN3:p.R1183Q, MAP2K4:p.R287H, MAPK8IP1:p.L217fs, MFSD5:p.R280Q, MUC16:p.R8606H, MYO6:p.D1180N, NAA25:p.S807Y, NBPF14:p.V44L, NRAS:p.Q61K, NRAS:p.G13R, PAX3:p.T424M, PGAM1:p.R240H, PHF3:p.R1410I, PIK3CA:p.R88Q, PIK3CA:p.E545K, PIK3CA:p.H1047R, PLXNA3:p.V14fs, POSTN:p.R508C, PTPRU:p.D1434N, PYGO2:p.Q150fs, RBBP7:p.E274K, SFPQ:p.R611Q, SGSM1:p.F1117L, SLC25A40:p.R96Q, SLC8A1:p.R431H, SLITRK3:p.S298L, SPATA22:p.S150L, SUN3:p.E128K, TGFBR1:p.S241L, TP53:p.R273H, TP53:p.R273C, TP53:p.R248W, TRPVS:p.R492H, USP40:p.S851L, VPS13C:p.D1359Y, ZBTB24:p.L607I, ZNF434:p.R306C, ZNF443:p.R301I, ZNF484:p.R138C, and ZNF770:p.S441P.

Referring to block 1032, in some embodiments, the tumor neoantigen and HLA allele are selected from the following neoantigen and HLA allele pairs: TP53 (R175H)-A*02:01, TP53 (Y220C)-A*02:01, and TP53 (R248W)-A*68:01.

Referring to block 1034, in some embodiments, the plurality of subjects includes subjects having a viral infection, and where the peptide of interest is an epitope derived from an antigen of the virus.

Referring to block 1036, in some embodiments, a clustering step is performed, wherein the first plurality of sequences is clustered into a first plurality of sequence groups based on sequence similarity between the respective sequences for the first TCR chain in the first plurality of sequences. Referring to block 1037, in some embodiments, a clustering step is performed, wherein the second plurality of sequences into a second plurality of sequence groups based on sequence similarity between the respective sequences for the second TCR chain in the second plurality of sequences.

Any suitable clustering method can be used to cluster the first plurality of sequences and the second plurality of sequences. In some embodiments, a two-step clustering approach is used in which an N x M matrix of CDR3 amino acid sequence and physicochemical properties is sorted into superclusters using the Faiss library, and the resulting embeddings are sorted with KMeans. A graph network of distances is then produced from these superclusters based on Hamming distances between length sorted CDR3 sequences. Final cluster assignments are made by applying Markov Clustering (MCL) to the network graph. In some embodiments, multidimensional scaling (MDS) to produce matrix representations of TCR CDR3 sequences that approximate BLOSUM62 physicochemical properties, such that the Euclidean distance between two sequences represented with MDS is equivalent to the Smith-Waterman alignment between the BLOSUM representations of those sequences. MDS vectors are pre-sorted on length, and the resulting superclusters are then sorted into subclusters using the Faiss library before clustering on Smith-Waterman distances between kmers. In some embodiments, a combination of global and local cluster analyses is used. Global distance is defined as sequence mismatches in CDR3 sequences differing at a given position according to a BLOSUM62 substitution matrix, having shared TRBV gene usage and identical length. Local distance is computed as a statistically significant kmer frequency enrichment in residues predicted to contact peptide-MHC, compared to a sample population. In some embodiments, the clustering method incorporates CDR3 and (optionally) V gene usage information, pre-sorting CDR3 sequences according to length and imposing a gap penalty for length mismatched CDR3s related by a single insertion. Alignment scores are computed for a subset of the CDR3 sequences using a BLOSUM62 substitution matrix, and output clusters are assigned based on a threshold alignment score. In some embodiments, the clustering method makes use of a BLOSUM62 mismatch distance between CDR1, CDR2, CDR2.5 (an MHC-facing loop), and CDR3 sequences. Non CDR3 sequences are inferred from a reference database, a gap penalty is applied to account for sequence insertions/deletions, and a combined similarity score is computed that assigns greater weighting to CDR3 sequences. The resulting distance matrix is then clustered, for example using a greedy hierarchical search.

Referring to block 1038, in some embodiments, the clustering of the first plurality of sequences and/or the clustering of the second plurality of sequences is based on at least CDR3 sequence similarity.

Referring to block 1040, in some embodiments, the clustering of the first plurality of sequences and/or the clustering of the second plurality of sequences is based on at least CDR3 length.

Referring to block 1042, in some embodiments, the clustering of the first plurality of sequences and/or the clustering of the second plurality of sequences is based on identical sequence length and sequence similarity.

Referring to block 1044, in some embodiments, the clustering of the first plurality of sequences and/or the clustering of the second plurality of sequences is based on identical sequence length and sequence similarity.

Referring to block 1046, in some embodiments, the clustering of the first plurality of sequences and/or the clustering of the second plurality of sequence is performed on at least 1 x 102, at least 1 x 103, at least 1 x 104, at least 1 x 105, or at least 1 x 106 sequences.

Referring to block 1048, in some embodiments, the respective sequences for the first TCR chain in the first plurality of sequence groups and the respective sequences for the second TCR chain in the second plurality of sequence groups are collectively representative of TCR sequences in the corresponding biological samples for at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95% or at least 99% of the plurality of subjects.

Referring to block 1050, in some embodiments, the first subset of the first plurality of sequence groups contains TCR α-chain sequences and the second subset of the second plurality of sequence groups contains TCR β-chain sequences.

Referring to block 1052, in some embodiments, the first subset of the first plurality of sequence groups contains TCR γ-chain sequences and the second subset of the second plurality of sequence groups contains TCR δ-chain sequences.

Referring to block 1054, in some embodiments, an identification step is performed, wherein a first subset of the first plurality of sequence groups containing sequences that are more prevalent in a first subset of the plurality of subjects that have both the peptide of interest and the HLA than in a second subset of the plurality of subjects that do not have both the peptide of interest and the HLA are identified. Referring to block 1055, in some embodiments, an identification step is performed, wherein a second subset of the second plurality of sequence groups containing sequences that are more prevalent in the first subset of the plurality of subjects that have the peptide of interest and the HLA than in the second subset of the plurality of subjects that do not have the peptide of interest and the HLA are identified.

In some embodiments, the plurality of subjects are cancer subjects, and first subset of the plurality of subject is responsive to a cancer treatment (e.g., does not exhibit a cancer progression event after treatment), and the second subset of the plurality of subject is unresponsive to the cancer treatment. Such an embodiment is useful for identify TCR chains and combinations that are may be enriched in the response cancer patient group.

Any suitable method can be used to identify the presence of the peptide of interest and HLA in a subject of the plurality of subjects. In some embodiments, the sequence read profile for each of the subject in the plurality of subject has a subject profile that includes nucleic acid sequence read data that is used to determine the presence of the peptide of interest and a particular HLA in a subject. Exemplary sequence reads include but are not limited to, tumor mRNA expression, germline DNA and/or tumor DNA data, or a subset of such data for each subject in the database. In some embodiments, presence of the peptide of interest (e.g., a neoantigen) in a subject is based on the presence of a threshold number of nucleic acid sequence reads of the peptide of interest in a sequence read profile of the subject. In some embodiments, presence of the peptide of interest in the subject is determined by a biomarker detection method including, for example, a fluorescence in situ hybridization (FISH) or immunohistochemical (IHC) detection method.

Any suitable method can be used to determine whether the first subset of the first plurality of sequence groups or second subset of the second plurality of sequence groups containing sequences are more prevalent in the first subset of the plurality of subjects that have the peptide of interest and the HLA than in the second subset of the plurality of subjects that do not have the peptide of interest and the HLA. Exemplary methods include but are not limited to: Fisher's exact p test, Barnard's exact test, Barnard's exact test, Boschloo's test, and Cochran-Mantel-Haenszel test.

Referring to block 1056, in some embodiments, the sequences of the first plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects.

Referring to block 1058, in some embodiments, the sequences of the second plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects.

Referring to block 1060, in some embodiments, the first threshold percentage is about 0.1%, about 0.5%, about 1%, about 5%, about 10%, about 15%, about 20%, about 25%, about 30%, about 35%, about 40%, about 45%, about 50%, about 60%, about 65%, about 70%, about 75%, about 80%, about 85%, about 90%, about 95% or about 99%.

Referring to block 1062, in some embodiments, the second threshold percentage is about 0.5%, about 1%, about 5%, about 10%, about 15%, about 20%, about 25%, about 30%, about 35%, about 40%, about 45%, or about 50%.

Referring to block 1064, in some embodiments, respective subjects in the first subset of the plurality of subjects and the second subset of the plurality of subjects are identified based on sequencing data from corresponding samples of cancerous tissues from the respective subjects.

Referring to block 1065, in some embodiments, an identification step is performed, wherein a plurality of TCR candidate pairs are identified, where each respective TCR candidate pair includes a respective sequence for the first TCR chain present in the first subset of the first plurality of sequence groups or a variant thereof and a respective sequence for the second TCR chain present in the second subset of the second plurality of sequence groups or a variant thereof.

Referring to block 1066, in some embodiments, the identifying includes screening for TCR pairs in which the respective sequence for the first TCR chain and the respective sequence for the second TCR chain are co-expressed in a threshold number of subjects in the first subset of the plurality of subjects.

Referring to block 1068, in some embodiments, the threshold number of subjects is at least 10, at least 25, at least 50, or at least 100 subjects.

Referring to block 1070, in some embodiments, the respective sequence for the first TCR chain includes a CDR3 sequence of the first TCR chain and the respective sequence for the second TCR chain includes a CDR3 sequence of the second TCR chain.

Referring to block 1072, in some embodiments, each respective TCR candidate pair includes a first TCR chain and a second TCR chain that is expressed in the same subject.

Referring to block 1074, in some embodiments, the first TCR chain is a TCR α-chain and the second TCR chain is a TCR β-chain.

Referring to block 1076, in some embodiments, the first TCR chain is a TCR γ-chain and the second TCR chain is a TCR δ-chain.

Referring to block 1077, in some embodiments, screen the plurality of TCR candidate pairs for the ability to bind the peptide of interest, thereby identifying a T cell receptor (TCR) that is capable of binding to a peptide of interest.

Referring to block 1078, in some embodiments, the screening includes expressing the plurality of TCR candidate pairs in a plurality of cells, where an individual cell of the plurality of cells expresses a TCR candidate pair of the plurality of TCR candidate pairs.

Referring to block 1080, in some embodiments, the screening step g) includes contacting the plurality of cells expressing the plurality of TCR candidate pairs with peptide of interest/MHC (pMHC) multimers and isolating cells that bind the pMHC multimers.

Referring to block 1082, in some embodiments the cells that bind the pMHC multimers are isolated using a cell sorting method.

Referring to block 1084, in some embodiments, the cell sorting method is selected from magnetic-activated cell sorting (MACS), fluorescence-activated cell sorting (FACS), and buoyancy-activated cell sorting.

Referring to block 1086, in some embodiments, the pMHC multimers are tetramers.

Referring to block 1088, in some embodiments, one or more TCR candidate pairs in the plurality of TCR candidate pairs includes a variant of a TCR α-chain that is present in the first subset of the first plurality of sequence groups and/or b) variant of a first TCR β -chain that is present in the second subset of the second plurality of sequence groups.

Referring to block 1090, in some embodiments, the variant TCR α-chain has the CDR3 sequence of a TCR α-chain that is present in the first subset of the first plurality of sequence groups.

Referring to block 1092, in some embodiments, the variant TCR α-chain does not have the CDR1 sequence and/or CDR2 sequence of the TCR α-chain that is present in the first subset of the first plurality of sequence groups.

Referring to block 1094, in some embodiments, the variant TCR β -chain has the CDR3 sequence of a TCR β -chain that is present in the second subset of the second plurality of sequence groups.

Referring to block 1096, in some embodiments, the variant β-chain does not have the CDR1 sequence and/or CDR2 sequence of the TCR β -chain that is present in the second subset of the second plurality of sequence groups.

Referring to block 1098, in some embodiments, the variant TCR α-chain is at least about 70%, at least about 80%, at least about 90%, at least about 95%, or at least about 98% identical to the TCR α-chain that is present in the first subset of the first plurality of sequence groups.

Referring to block 1100, in some embodiments, the variant TCR β-chain is at least about 70%, at least about 80%, at least about 90%, at least about 95%, or at least about 98% identical to the TCR β-chain that is present in the second subset of the second plurality of sequence groups.

Referring to block 1102, in some embodiments, one or more TCR candidate pairs of the plurality of TCR candidate pairs includes a variant of a TCR γ-chain that is present in the first subset of the first plurality of sequence groups and/or variant of a first TCR δ -chain that is present in the second subset of the second plurality of sequence groups.

Referring to block 1104, in some embodiments, the variant TCR γ-chain has the CDR3 sequence of a TCR α-chain that is present in the first subset of the first plurality of sequence groups.

Referring to block 1106, in some embodiments, he variant TCR γ-chain does not have the CDR1 sequence and/or CDR2 sequence of the TCR γ-chain that is present in the first subset of the first plurality of sequence groups.

Referring to block 1108, in some embodiments, the variant TCR δ-chain has the CDR3 sequence of a TCR β -chain that is present in the second subset of the second plurality of sequence groups.

Referring to block 1110, in some embodiments, the variant δ-chain does not have the CDR1 sequence and/or CDR2 sequence of the TCR δ -chain that is present in the second subset of the second plurality of sequence groups.

Referring to block 1112, in some embodiments, the variant TCR γ-chain is at least about 70%, at least about 80%, at least about 90%, at least about 95%, or at least about 98% identical to the TCR γ-chain that is present in the first subset of the first plurality of sequence groups.

Referring to block 1114, in some embodiments, the variant TCR δ -chain is at least about 70%, at least about 80%, at least about 90%, at least about 95%, or at least about 98% identical to the TCR δ-chain that is present in the second subset of the second plurality of sequence groups.

Referring to block 1116, in some embodiments, sequence candidate TCRs that are determined to bind to a peptide of interest in complex with the HLA.

Referring to block 1118, in some embodiments, the screening includes in silico modeling of an interaction between respective TCR candidate pairs and the peptide of interest.

### VII. TCR Compositions

In another aspect, provided are TCR compositions comprising TCRs produced by the subject methods described herein.

In some embodiments, the composition comprises non-naturally occuring T-cells that are engineered to express a TCR identified using the subject methods. In some embodiments, the T-cell includes one or more expression vectors encoding the TCR chains (e.g., a TCR α- and β-chain of a TCR identified using the subject methods). In some embodiments, the T-cell includes a first expression vector that includes a nucleic acid encoding a TCR α-chain, and a second expression vector that includes a nucleic acid encoding a TCR β-chain. Such nucleic acids can be introduced into T-cells using any suitable technique known in the art including, for example, know transfection techniques (see, e.g., Robbins et al., J Immunol. 180:6116-6131 (2008)) and transduction e.g., retroviral transduction, lentiviral transduction, and adenoviral transduction.

Also provided herein are soluble TCRs produced by the subject methods described herein. Soluble TCRs are useful, for example, for delivering detectable labels or therapeutic agents to the antigen presenting cells and tissues containing the antigen presenting cells. In some embodiments, the soluble TCR is attached to a detectable label. Exemplary detectable labels include, but are not limited to, fluorescent labels, nucleic acid probes, radiolabels, enzymes, and contrast reagents. In some embodiments, the TCR is attached to a therapeutic agent.

In another aspect, provided herein are pharmaceutical compositions that include a non-naturally occuring T-cell or a soluble TCR described herein, and a pharmaceutical carrier or excipients.

### VIII. Methods of Treatment

In another aspect, provided herein are methods of treatment comprising administering a pharmaceutical composition to a subject in need thereof, wherein the pharmaceutical composition comprising a subject engineered T-cell or TCR as described herein. T-cells that are engineered to express one or more TCRs using the subject methods described herein are useful, for example, in adoptive therapies for treatment of diseases (e.g., a cancer or a viral disease). Suitable adoptive therapy methods are described, for example, in Rosenberg et al., Nat Rev Cancer 8(4):299-308 (2008). The pharmaceutical composition may be adapted for administration by any appropriate route, preferably a parenteral (including subcutaneous, intramuscular, or preferably intravenous) route. Such compositions may be prepared by any method known in the art of pharmacy, for example by mixing the active ingredient with the carrier(s) or excipient(s) under sterile conditions.

In some embodiments, a therapeutically effective amount of the engineered T-cells are administered to a patient in need thereof. A therapeutically effective dose is an amount of cells used in adoptive transfer that is capable of producing a clinically desirable result (i.e., a sufficient amount to induce or enhance a specific T cell immune response in a statistically significant manner) in a treated human or non-human mammal. As is well known in the medical arts, the dosage for any one patient depends upon many factors, including the patient's size, weight, body surface area, age, the particular therapy to be administered, sex, time and route of administration, general health, and other drugs being administered concurrently.

Pharmaceutical compositions may be administered in a manner appropriate to the disease or condition to be treated (or prevented) as determined by persons skilled in the medical art. An appropriate dose and a suitable duration and frequency of administration of the compositions will be determined by such factors as the health condition of the patient, size of the patient (i.e., weight, mass, or body area), the type and severity of the patient's disease, the particular form of the active ingredient, and the method of administration. In general, an appropriate dose and treatment regimen provide the composition(s) in an amount sufficient to provide therapeutic and/or prophylactic benefit (such as described herein, including an improved clinical outcome, such as more frequent complete or partial remissions, or longer disease-free and/or overall survival, or a lessening of symptom severity). For prophylactic use, a dose should be sufficient to prevent, delay the onset of, or diminish the severity of a disease associated with disease or disorder. Prophylactic benefit of the immunogenic compositions administered according to the methods described herein can be determined by performing pre-clinical (including in vitro and in vivo animal studies) and clinical studies and analyzing data obtained therefrom by appropriate statistical, biological, and clinical methods and techniques, all of which can readily be practiced by a person skilled in the art.

### EXAMPLES

Examples are provided below to illustrate the present invention. These examples are not meant to constrain the present invention to any particular application or theory of operation.

### Example 1: TCR Database

Specimens were sequenced from several thousand cancer patients. The associated RNA sequence data includes nucleic acid sequences from immune cells in those cancer specimens (tumor infiltrating lymphocytes, especially T cells and B cells), as well as amino acid sequences derived from such nucleic acid sequences. The sequencing probes were designed specifically to capture sequence reads corresponding to the T cell receptor and B cell receptor genes, especially the CDR3 regions of those genes, which is the region with the largest influence over whether the receptor can bind to a particular antigen.

Each recorded TCR sequence includes the CDR3 region and associated metadata including a classification for the V, D (if present), J, and C regions of the receptor, as well as an alpha or beta chain designation. T cell receptor sequences, associated TCR metadata, and patient data were put into a database for further analysis.

### Example 2: Identification of Candidate TCRs that bind Neoantigen/HLA allele

Candidate TCRs were determined for neoantigen TP53p.R175H, with associated HLA A*02:01. The TCR database was filtered to select patients having an HLA class associated with the neoantigen (HLA A*02:01). Optionally, the analysis could further filter patients by clinical data (for example, by cancer type, HLA LOH status, tumor mutation burden (TMB), microsatellite instability (MSI), PD-L1 other biomarker, treatment history, etc.). For the first neoantigen, a TP53 variant (TP53p.R175H, with associated HLA A*02:01) was selected.

The filtered patients were categorized into two groups based on the patient DNA data. Patients who had the DNA variant in their cancer cells corresponding with the selected TP53p.R175H neoantigen, were included in a positive control patient group. Patients without the neoantigen in their cancer cells were added to a negative control patient group.

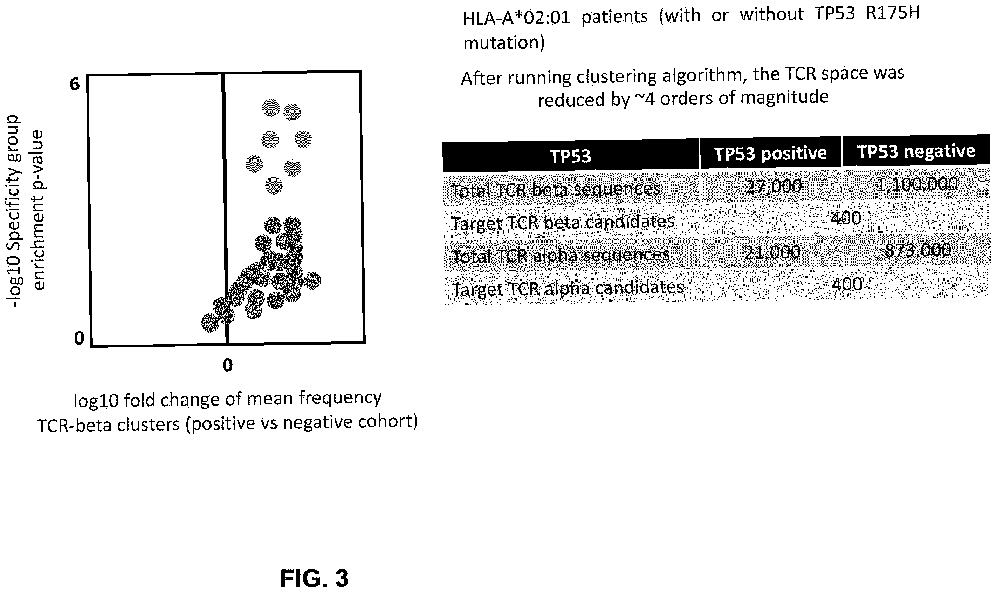

A keyword clustering algorithm was subsequently used to organize the TCR sequences for each patient group into clusters based on similarities among the CDR3 sequences. Negative control clusters were compared with positive control clusters to determine TCR sequences that are prevalent in the positive control group but less prevalent or absent in the negative control group. This yielded 557 alpha sequences and 511 beta sequences, which were subsequently narrowed down to the 400 most enriched sequences for each group (400 α-chains and 400 β-chains, See Figure 3).

For each CDR3 region sequence identified, the sequence was converted into a full alpha or beta chain sequence by adding to the recorded partial V region sequence with the remainder of the most similar known reference V region sequence. Similar replacements were performed for partial J region sequences. Optionally, replacements for any constant (C) region sequences can be performed. Such constructed alpha or beta chain each have the same CDR3 .

### Example 3: TCR Mapping

For each of the 160,000 candidate TCR α-chains and β-chain sequence combinations identified in the computational analysis, lentiviruses were genetically engineered to carry a lentiviral vector encoding the TCR alpha beta sequence combination. For each combination, a line of T cells (Jurkat) was infected with multiple copies of the corresponding lentivirus, creating a clone of T cells that expressed the particular TCR. The T cell line (Jurkat, Clone E6-1), does not naturally express a TCR.

A lentiviral vector was constructed to accept one alpha chain sequence and one beta chain sequence. The lentiviral vector included a constitutively active EF-1 alpha promoter. In addition the lentiviral vector included constant regions for each chain, each having a signal peptide that would facilitate localization of the candidate TCR to the cell membrane. Between the vector's placeholders for the α-chain and β-chain sequence, the vector had a cleavage signal that would cause the α-chain and β-chain c to separate after being expressed as one polypeptide in the T cell. The vector was also designed to allow T cells to grow in the presence of the antibiotic puromycin only if they are expressing the vector, to facilitate screening and elimination of any T cells that were not successfully infected.

The lentiviral vectors were transfected into HEK293T cells and the cells generated infectious versions of the lentiviruses. Those infectious lentiviral particles were mixed with T cells (e.g., Jurkat, Clone E6-1). The cells were screened to eliminate cells that were not infected, cells without the vector or TCR sequence. Screening included staining surface TCR molecules (specifically the constant region of the beta chain) and sorting using FACS, then puromycin antibiotic selection.

Tetramer staining was used to test each candidate TCR to determine whether it binds to the neoantigen peptide when loaded in the HLA associated with that neoantigen. Each tetramer contained four copies of the peptide (9 amino acids long) generated by the neoantigen, the HLA associated with the antigen, and a fluorescent marker (APC). FACS was used to isolate any T cells that recognized the HLA-loaded neoantigen, demonstrated by the cell binding to a fluorescent tetramer.

Reactive T cells identified by tetramer staining were sequenced using long read nanopore sequencing (PacBio) to determine/confirm the full TCR sequence that recognized the antigen.

Validation will include specificity testing to determine whether TCR leads are specific to the selected neoantigen and associated HLA (instead of having the ability to bind/recognize multiple antigens, and potentially cause off-target effects). Validation experiments include 1) monitoring T cell proliferation (should only occur with neoantigen and associated HLA, not with other HLAs or other peptides), 2) ELISPOT assay to determine which peptides can bind to the TCR and search database of known human peptides to predict whether TCR will react to healthy human cells, 3) co-culturing the reactive T cells with organoids and assessing whether the T cells specifically kill organoid cells that express the neoantigen (and the HLA associated with that antigen).

### Example 4: Interrogating Real World Tumor-Infiltrating T-cell Repertoires to Identify Antigen Enriched TCRs in a Large Pan-cancer Clinical Cohort

### Background

TCR repertoire profiling can provide a useful window into the complex interactions between tumor cells and infiltrating lymphocytes. Despite recent advances in repertoire sequencing methods, the characterization of tumor-infiltrating T-cell repertoires has been limited by access to large, real-world patient cohorts with genomic, TCR, and clinical profiling. In this study, identification of public TCRs associated with HLA-specific neoantigens and viral epitopes was performed by analyzing a large, real-world, clinic genomic database of over 130K patients, with T-cell repertoire data covering a diverse landscape of HLA genotypes and tumor neoantigens.

### Methods

TCR sequences were detected using a hybrid-capture whole exome RNA assay optimized with probes for capture of TCR genes (see, e.g., US Patent No. 11,414,700). Repertoire profiling reads were aligned, assembled, and annotated against IMGT reference sequences. Neoantigens were identified from paired samples using a targeted DNA panel covering 648 cancer-associated genes. Human papillomavirus (HPV) status was determined using a panel of probes to E6 and E7 genes in HPV16, 18 and 33 and captured by both the RNA and DNA assays.

### Results

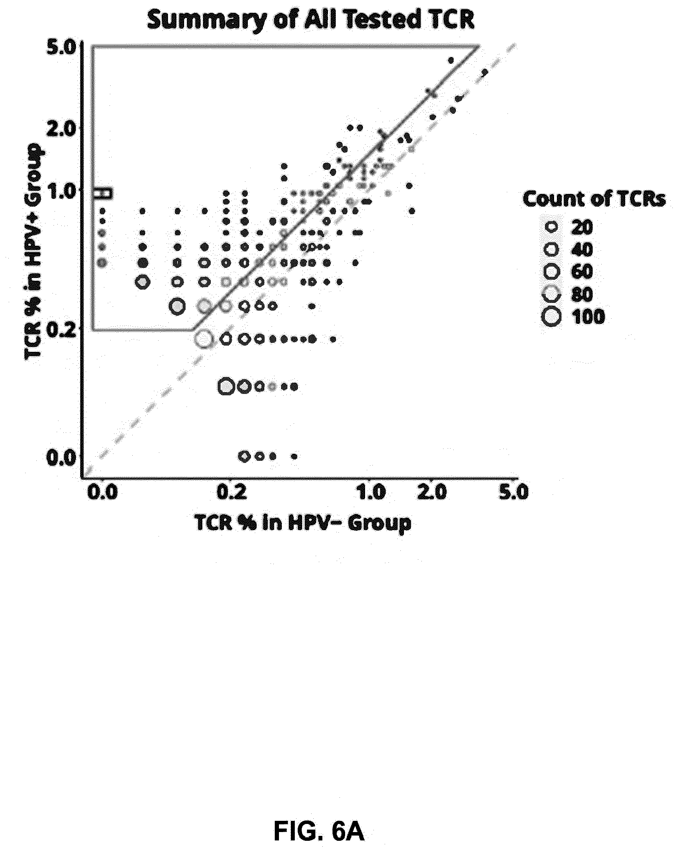

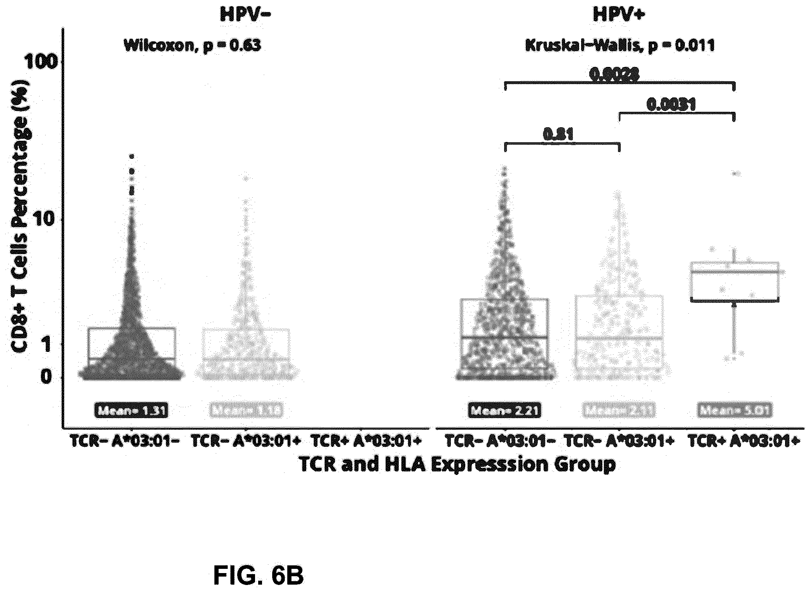

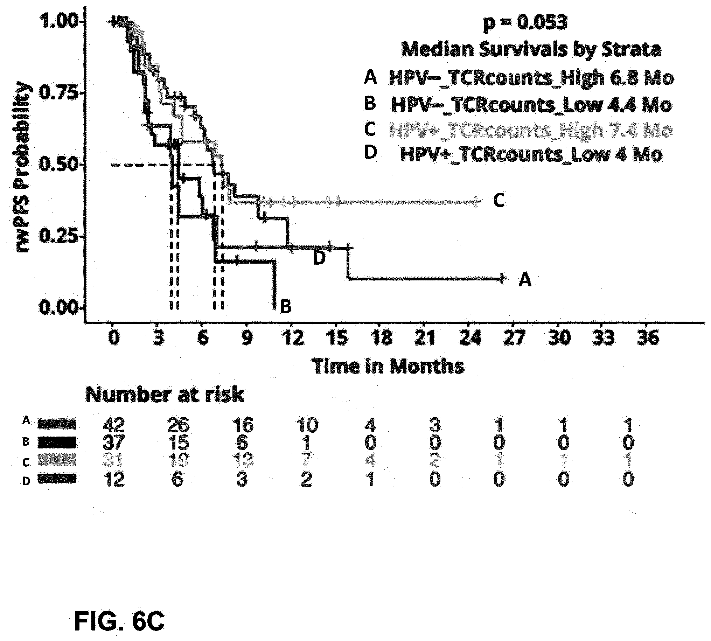

Investigation of intermediate public TCRs, defined as TCRs shared among 10 < N < 1000 patients, revealed TCRs that were co-occurring with multiple neoantigens, including *KRAS, TP53,* and *EGFR* (Fisher adjusted p<0.001). *KRAS* Q61H-associated TCRs were enriched within subjects with A*01:01, consistent with peptides predicted to bind and present by MHC class I allele. Expanding beyond neoantigens to viral epitopes, integration of HPV status in parallel with repertoire data revealed TCRs that were significantly enriched within HPV positive tumors in HNSCC and CESC (P < 0.05, Fisher Exact Test), but not the inverse, consistent with antigen selection (Figure 6). Figure 6 provides the summary of a study of the characterization of HPV associated TCRs and the associated tumor microenvironment. 1151 HPV+ and 2118 HPV- tumors were characterized across cervical and head and neck (Figure 6A)). TCRβs were tested for association with HPV. Enriched TCRβs were specifically seen in HPV+ tumors (Fisher p<0.05 and odds ratio >1). Interrogation of one TCRβ (black box in Figure 6(A)), specific to A*03:01 demonstrated that presence of this TCRβ is seen in more CD8 infiltrated tumors (Figure 6B)). Within HNSCC patients receiving immunotherapy, pre-treatment high total TCRβ counts showed a longer median rwPFS compared to low TCRβ abundance, independent of HPV status (Figure 6(C)).

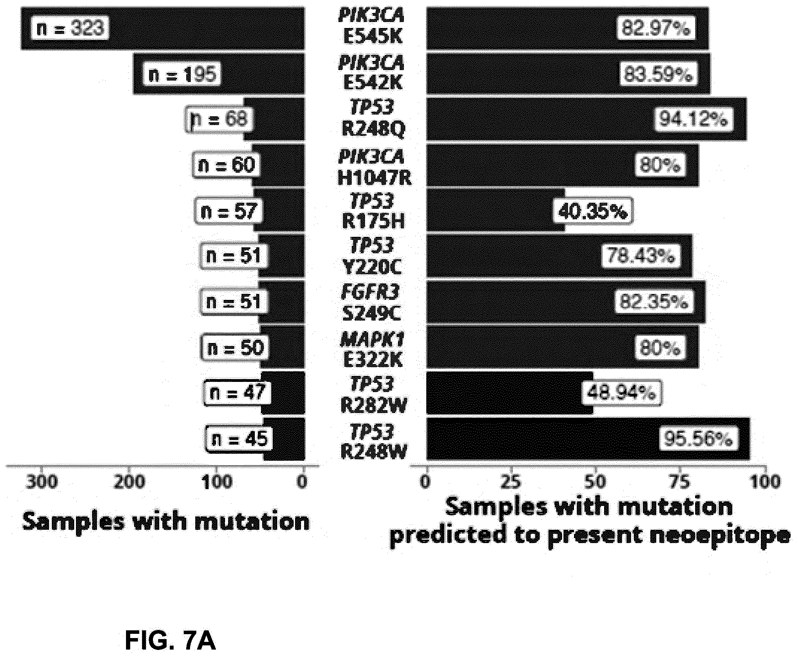

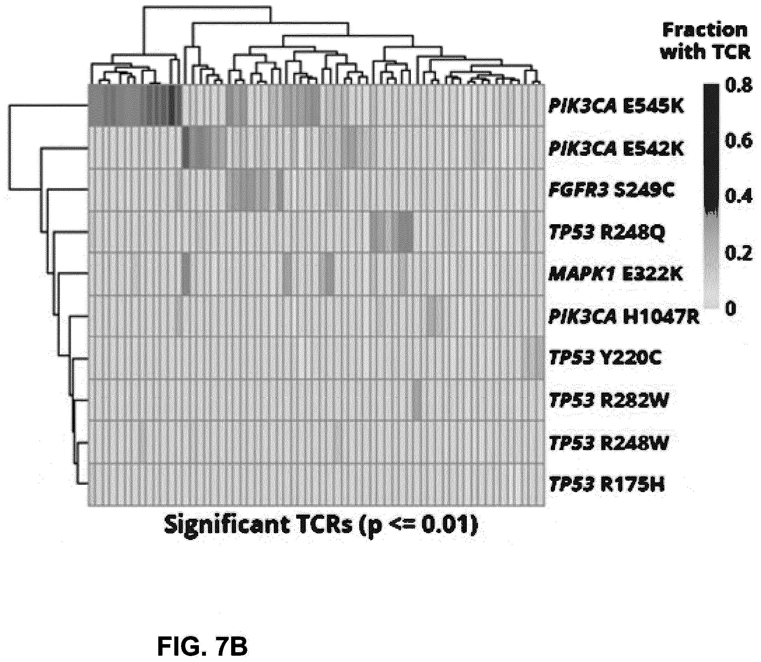

Similar to *KRAS* neoantigen enriched TCRs, these TCRs were associated with specific HLA class I alleles (Figure 7). Figure 7 provides a summary of a study of the identification of neoantigen specific TCRs. The most prevalent pathogenic neoantigens were identified within the cohort of cervical and head and neck cancer patients along with what fraction present those neoantigens, integrating HLA typing, demonstrating variable prevalence of predicted immunogenicity (Figure 7(A)). Fisher exact tests were performed on all TCRβs present in at least 5 individuals to identify TCRβs enriched within specific neoantigen contexts (Figure 7(B)). PIK3CA E542K and E545K have among the highest number of associated TCRβs.

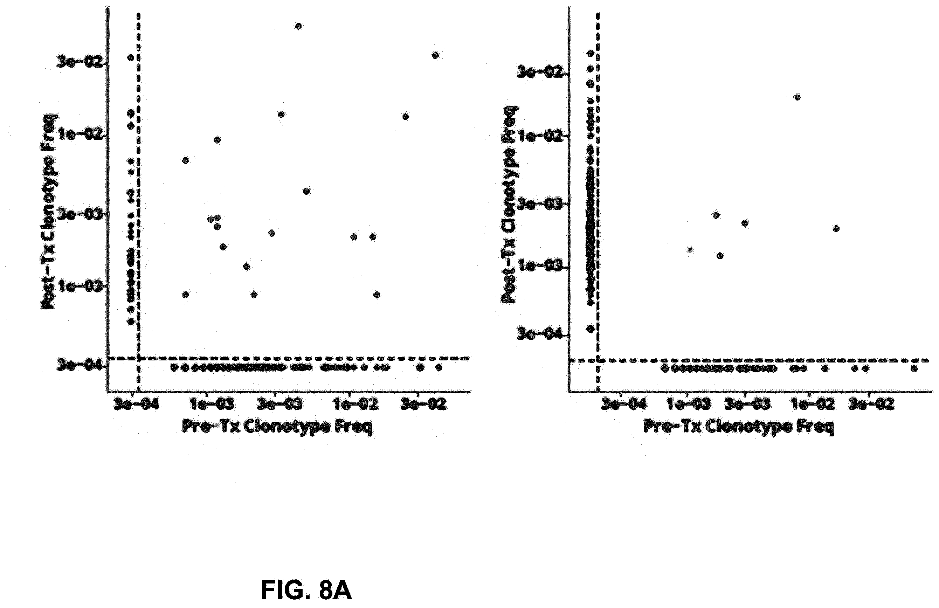

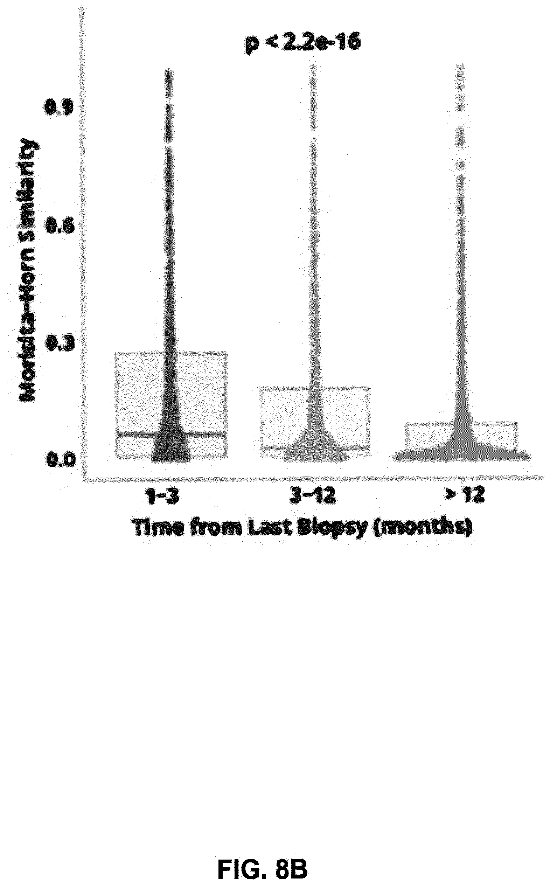

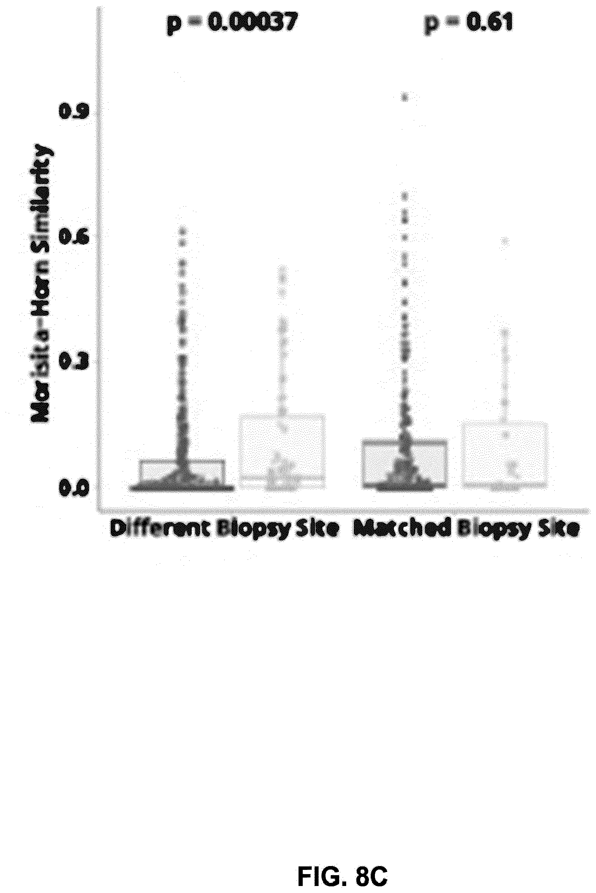

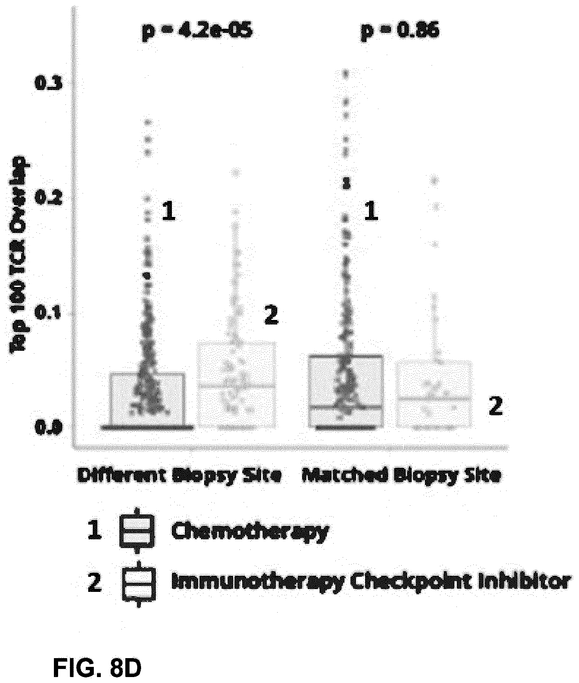

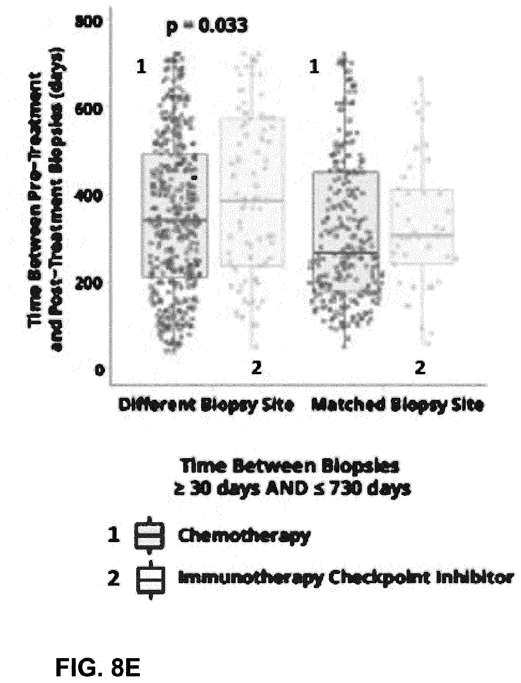

Further, patient TCR repertoire can change over time due to both temporal and treatment (e.g., chemotherapy vs. immunotherapy) changes (Figure 8). Comparisons of samples over time can lend insights into repertoire dynamics based on regimen and biopsy site. Figure 8(A) provides an illustrative example of two patients' repertoires over time. As shown in Figure 8(B) Repertoire similarity decreases over time due to both temporal and treatment associated changes. Comparison of chemotherapy and immunotherapy associated repertoire changes, demonstrated that treatment with immunotherapy results in a more similar repertoire when sampled from distinct biopsy sites (Figure 8(C)). This result was consistent when quantifying just the top 100 most frequent clones in each sample (Figure 8(D)). Further, sample timing did not differ within these groups in the direction that would bias this result (Figure 8(E)).

### Conclusions

By incorporating TCR repertoire profiling into a high-volume clinical genomic sequencing program, a rich, multi-modal resource was developed for studying the complex tumor-immune interaction. Analysis of this resource - encompassing a diverse collection of cancer types, HLA genotypes, and mutational/viral contexts - revealed a subset of TCRs enriched with allele-specific neo-antigens. This dataset is a valuable resource for TCR therapeutic discovery, and can help identify naturally occuring TCRs that may minimize on-target, off-tumor toxicity.

The examples set forth above are provided to give those of ordinary skill in the art a complete disclosure and description of how to make and use the embodiments of the compositions, systems and methods of the invention, and are not intended to limit the scope of what the inventors regard as their invention. Modifications of the above-described modes for carrying out the invention that are obvious to persons of skill in the art are intended to be within the scope of the following claims. All patents and publications mentioned in the specification are indicative of the levels of skill of those skilled in the art to which the invention pertains.

All headings and section designations are used for clarity and reference purposes only and are not to be considered limiting in any way. For example, those of skill in the art will appreciate the usefulness of combining various aspects from different headings and sections as appropriate according to the spirit and scope of the invention described herein.

All references cited herein are hereby incorporated by reference herein in their entireties and for all purposes to the same extent as if each individual publication or patent or patent application was specifically and individually indicated to be incorporated by reference in its entirety for all purposes.

Many modifications and variations of this application can be made without departing from its spirit and scope, as will be apparent to those skilled in the art. The specific embodiments and examples described herein are offered by way of example only, and the application is to be limited only by the terms of the appended claims, along with the full scope of equivalents to which the claims are entitled.

### EMBODIMENTS

Embodiment 1 - A method of identifying a T cell receptor (TCR) that is capable of binding to a peptide of interest in complex with a human leukocyte antigen (HLA), the method comprising: at a computer system having one or more processors, and memory storing one or more programs for execution by the one or more processors: a) identifying a first plurality of sequences for a first T-Cell Receptor (TCR) chain and a second plurality of sequences for a second TCR chain from a plurality of sequence read profiles, wherein each respective sequence read profile in the plurality of sequence read profiles comprises corresponding sequence reads for amino acids in a biological sample from a respective subject in a plurality of subjects, and wherein the first TCR chain and second TCR chain are a TCR α-chain and a TCR-β-chain, respectively, or a TCR γ-chain and a TCR δ-chain, respectively; b) clustering the first plurality of sequences into a first plurality of sequence groups based on sequence similarity between the respective sequences for the first TCR chain in the first plurality of sequences; c) clustering the second plurality of sequences into a second plurality of sequence groups based on sequence similarity between the respective sequences for the second TCR chain in the second plurality of sequences; d) identifying a first subset of the first plurality of sequence groups containing sequences that are more prevalent in a first subset of the plurality of subjects that have both the peptide of interest and the HLA than in a second subset of the plurality of subjects that do not have both the peptide of interest and the HLA; e) identifying a second subset of the second plurality of sequence groups containing sequences that are more prevalent in the first subset of the plurality of subjects that have the peptide of interest and the HLA than in the second subset of the plurality of subjects that do not have the peptide of interest and the HLA; f) identifying a plurality of TCR candidate pairs, wherein each respective TCR candidate pair comprises a respective sequence for the first TCR chain present in the first subset of the first plurality of sequence groups or a variant thereof and a respective sequence for the second TCR chain present in the second subset of the second plurality of sequence groups or a variant thereof, and g) screening the plurality of TCR candidate pairs for the ability to bind the peptide of interest, thereby identifying a T cell receptor (TCR) that is capable of binding to a peptide of interest.

Embodiment 2 - The method of embodiment 1, wherein the first TCR chain is a TCR α-chain and the second TCR chain is a TCR β-chain.

Embodiment 3 - The method of embodiment 1, wherein the first TCR chain is a TCR γ-chain and the second TCR chain is a TCR δ-chain.

Embodiment 4- The method according to any one of embodiments 1 to 3 wherein the plurality of sequence read profiles comprises at least 500, at least 1,000, at least 5,000, at least 10,000, at least 20,000, at least 50,000, at least 100,000, at least 500,000, at least 1 million, at least 5 million, or at least 10 million sequence read profiles.

Embodiment 5 - The method according to any one of embodiments 1 to 4, wherein the corresponding sequence reads for the amino acids in the biological sample from the respective subject are sequence reads from a cancerous tissue of the respective subject.

Embodiment 6- The method according to any one of embodiments 1 to 5, wherein the plurality of subjects comprises cancer subjects.

Embodiment 7 - The method according to embodiment 6, wherein the cancer is selected from urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer.

Embodiment 8 - The method according to embodiment 6 or 7, wherein the peptide of interest is an epitope derived from a tumor-associated antigen, a tumor-specific antigen, or a neoantigen.

Embodiment 9 - The method according to any one of embodiments 6 to 8, wherein the cancer subjects have undergone a treatment for the cancer.

Embodiment 10 - The method of embodiment 9, wherein the treatment is selected from surgery, chemotherapy, immunotherapy, bone marrow transplant, immunotherapy, hormone therapy, targeted drug therapy, cryoablation, radiofrequency ablation, and combinations thereof.

Embodiment 11 - The method of embodiment 10, wherein the immunotherapy is selected from an antibody therapy, a cytokine therapy, an oncolytic virus therapy, an adoptive cell transplant therapy, a cancer vaccine or combinations thereof.

Embodiment 12 - The method of embodiment 10, wherein the immunotherapy is an antibody therapy.

Embodiment 13 - The method of embodiment 12, wherein the antibody therapy is a human checkpoint inhibitor therapy, and wherein the human checkpoint is selected from one of the following: PD-1, PD-L1, CTLA-4, LAG3, TIM-3, B7H3, B7H4, A2aR, CD73, NIKG2A, PVRIG/PVRL2, CEACAM1, CECAM 5/6, FAK, CCL2/CCR2, LIF, CD47/SIRPα, CSF-1, IL-1, IL-1R3, IL-8, SEMA4D, Ang-2, CLEVER-1, Axl, and phosphatidylserine.

Embodiment 14 - The method according to any one of embodiments 6 to 13, wherein the plurality of subjects do not exhibit tumor cell loss of heterozygosity.

Embodiment 15 - The method according to any one of embodiments 6 to 14, wherein the peptide of interest is derived from a tumor neoantigen selected from KRAS:p.G12D, BRAF:p.V600E, KRAS:p.G12V, ACVR2A:p.K435fs, GRB14:p.KKK295del, SEC63:p.L532fs, TGFBR2:p.E125fs, ATR:p.K771fs, ICAl:p.N204fs, KRAS:p.G12C, TP53:p.R175H, ABCA8:p.R842Q, ACTL7B:p.R354H, ACVR2A:p.K435fs, AIM2:p.K340fs, ALG2:p.S302Y, ANKIB1:p.K144fs, ARSG:p.V131I, ATP10D:p.R311H, AXIN2:p.W663fs, C5orf30:p.D4N, CACNG3:p.V134I, CASP5:p.K78fs, CC2D2A:p.R1284C, CDH10:p.E349K, DNMTI:p.E432K, DOCK2:p.G170R, DOCKS:p.E177K, EGR2:p.R390H, ERBB3:p.V104M, FAM135B:p.R884H, FBXW7:p.R505C, FBXW7:p.R465H, FHDC1:p.R254W, FOXL1:p.N89K, HCN4:p.R525H, HLA-DMA:p.E84K, HTR3B:p.R236C, ITGA4:p.T673M, KIF18A:p.R17C, KIF20B:p.E991K, KLHL5:p.R326C, KRAS:p.A146T, KRAS:p.G13D, LPHN3:p.R1183Q, MAP2K4:p.R287H, MAPK8IP1:p.L217fs, MFSD5:p.R280Q, MUC16:p.R8606H, MYO6:p.D1180N, NAA25:p.S807Y, NBPF14:p.V44L, NRAS:p.Q61K, NRAS:p.G13R, PAX3:p.T424M, PGAM1:p.R240H, PHF3:p.R1410I, PIK3CA:p.R88Q, PIK3CA:p.E545K, PIK3CA:p.H1047R, PLXNA3:p.V14fs, POSTN:p.R508C, PTPRU:p.D1434N, PYGO2:p.Q150fs, RBBP7:p.E274K, SFPQ:p.R611Q, SGSM1:p.F1117L, SLC25A40:p.R96Q, SLC8A1:p.R431H, SLITRK3:p.S298L, SPATA22:p.S150L, SUN3:p.E128K, TGFBR1:p.S241L, TP53:p.R273H, TP53:p.R273C, TP53:p.R248W, TRPVS:p.R492H, USP40:p.S851L, VPS13C:p.D1359Y, ZBTB24:p.L607I, ZNF434:p.R306C, ZNF443:p.R301I, ZNF484:p.R138C, and ZNF770:p.S441P.

Embodiment 16 - The method of embodiment 15, wherein the tumor neoantigen and HLA allele are selected from the following neoantigen and HLA allele pairs: TP53 (R175H)-A*02:01, TP53 (Y220C)-A*02:01, and TP53 (R248W)-A*68:01.

Embodiment 17 - The method according to any one of embodiments 1 to 5, wherein the plurality of subjects comprises subjects having a viral infection, and wherein the peptide of interest is an epitope derived from an antigen of the virus.

Embodiment 18 - The method according to any one of embodiments 1 to 17, wherein the clustering of the first plurality of sequences and/or the clustering of the second plurality of sequences is based on at least CDR3 sequence similarity.

Embodiment 19 - The method according to any one of embodiments 1 to 17, wherein the clustering of the first plurality of sequences and/or the clustering of the second plurality of sequences is based on at least CDR3 length.

Embodiment 20 - The method according to any one of embodiments 1 to 19, wherein the clustering of the first plurality of sequences and/or the clustering of the second plurality of sequences is based on identical sequence length and sequence similarity.

Embodiment 21 - The method according to any one of embodiments 1 to 20, wherein the clustering of the first plurality of sequences and/or the clustering of the second plurality of sequence is performed on at least 1 × 10², at least 1 × 10³, at least 1 × 10⁴, at least 1 × 10⁵, or at least 1 × 10⁶ sequences.

Embodiment 22 - The method v according to any one of embodiments 1 to 21, wherein the respective sequences for the first TCR chain in the first plurality of sequence groups and the respective sequences for the second TCR chain in the second plurality of sequence groups are collectively representative of TCR sequences in the corresponding biological samples for at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95% or at least 99% of the plurality of subjects.

Embodiment 23 - The method according to any one of embodiments 1 to 22, wherein the first subset of the first plurality of sequence groups contains TCR α-chain sequences and the second subset of the second plurality of sequence groups contains TCR β-chain sequences.

Embodiment 24- The according to any one of embodiments 1 to 23, wherein the first subset of the first plurality of sequence groups contains TCR γ-chain sequences and the second subset of the second plurality of sequence groups contains TCR δ-chain sequences.

Embodiment 25 - The method according to any one of embodiments 1 to 24, where sequences of the first plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects.

Embodiment 26 - The method according to any one of embodiments 1 to 25, where sequences of the second plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects.

Embodiment 27 - The method according to embodiment 25 or 26, wherein the first threshold percentage is about 0.1%, about 0.5%, about 1%, about 5%, about 10%, about 15%, about 20%, about 25%, about 30%, about 35%, about 40%, about 45%, about 50%, about 60%, about 65%, about 70%, about 75%, about 80%, about 85%, about 90%, about 95% or about 99%.

Embodiment 28 - The method according to embodiment 25 or 26, wherein the second threshold percentage is about 0.5%, about 1%, about 5%, about 10%, about 15%, about 20%, about 25%, about 30%, about 35%, about 40%, about 45%, or about 50%.

Embodiment 29 - The method according to any one of embodiments 1 to 28, wherein respective subjects in the first subset of the plurality of subjects and the second subset of the plurality of subjects are identified based on sequencing data from corresponding samples of cancerous tissues from the respective subjects.

Embodiment 30 - The method according to any one of embodiments 1 to 29, wherein the identifying f) comprises screening for TCR pairs in which the respective sequence for the first TCR chain and the respective sequence for the second TCR chain are co-expressed in a threshold number of subjects in the first subset of the plurality of subjects.

Embodiment 31 - The method of embodiment 30, wherein the threshold number of subjects is at least 10, at least 25, at least 50, or at least 100 subjects.

Embodiment 32 -The method of any one of the proceeding embodiments, wherein the respective sequence for the first TCR chain comprises a CDR3 sequence of the first TCR chain and the respective sequence for the second TCR chain comprises a CDR3 sequence of the second TCR chain.

Embodiment 33 - The method according to any one of embodiments 1-33, wherein each respective TCR candidate pair comprises a first TCR chain and a second TCR chain that is expressed in the same subject.

Embodiment 34 - The method of embodiment 33, wherein the first TCR chain is a TCR α-chain and the second TCR chain is a TCR β-chain.

Embodiment 35- The method of embodiment 33, wherein the first TCR chain is a TCR γ-chain and the second TCR chain is a TCR δ-chain.

Embodiment 36 - The method according to any one of embodiments 1-35, wherein the screening step g) comprises expressing the plurality of TCR candidate pairs in a plurality of cells, wherein an individual cell of the plurality of cells express a TCR candidate pair of the plurality of TCR candidate pairs.

Embodiment 37 - The method of embodiment 36, wherein the screening step g) comprises contacting the plurality of cells expressing the plurality of TCR candidate pairs with peptide of interest/MHC (pMHC) multimers and isolating cells that bind the pMHC multimers.

Embodiment 38 - The method of embodiment 37, wherein the cells that bind the pMHC multimers are isolated using a cell sorting method.

Embodiment 39 - The method of embodiment 38, wherein the cell sorting method is selected from magnetic-activated cell sorting (MACS), fluorescence-activated cell sorting (FACS), and buoyancy-activated cell sorting.

Embodiment 40 - The method of according to any one of embodiments 37 to 39, where the pMHC multimers are tetramers.

Embodiment 41 - The method according to any one of embodiments 1-40, wherein one or more TCR candidate pairs in the plurality of TCR candidate pairs comprises: a) a variant of a TCR α-chain that is present in the first subset of the first plurality of sequence groups; and/or b) variant of a first TCR β -chain that is present in the second subset of the second plurality of sequence groups.

Embodiment 42 - The method of embodiment 41, wherein the variant TCR α-chain has the CDR3 sequence of a TCR α-chain that is present in the first subset of the first plurality of sequence groups.

Embodiment 43 -The method of embodiment 42, where the variant TCR α-chain does not have the CDR1 sequence and/or CDR2 sequence of the TCR α-chain that is present in the first subset of the first plurality of sequence groups.

Embodiment 44 - The method of any one of embodiments 41 to 43, wherein the variant TCR β -chain has the CDR3 sequence of a TCR β -chain that is present in the second subset of the second plurality of sequence groups.

Embodiment 45 - The method of embodiment 44 wherein the variant β-chain does not have the CDR1 sequence and/or CDR2 sequence of the TCR β -chain that is present in the second subset of the second plurality of sequence groups.

Embodiment 46 - The method according to any one of embodiments 41 to 45, wherein the variant TCR α-chain is at least about 70%, at least about 80%, at least about 90%, at least about 95%, or at least about 98% identical to the TCR α-chain that is present in the first subset of the first plurality of sequence groups.

Embodiment 47 - The method according to any one of embodiment 41 to 46, wherein the variant TCR β-chain is at least about 70%, at least about 80%, at least about 90%, at least about 95%, or at least about 98% identical to the TCR β-chain that is present in the second subset of the second plurality of sequence groups.

Embodiment 48 - The method of any one of embodiments 1 to 40, wherein one or more TCR candidate pairs of the plurality of TCR candidate pairs comprises: a) a variant of a TCR γ-chain that is present in the first subset of the first plurality of sequence groups; and/or b) variant of a first TCR δ -chain that is present in the second subset of the second plurality of sequence groups.

Embodiment 49 - The method of embodiment 48, wherein the variant TCR γ-chain has the CDR3 sequence of a TCR α-chain that is present in the first subset of the first plurality of sequence groups.

Embodiment 50 - The method of embodiment 49, where the variant TCR γ-chain does not have the CDR1 sequence and/or CDR2 sequence of the TCR γ-chain that is present in the first subset of the first plurality of sequence groups.

Embodiment 51 -The method of any one of embodiments 48 to 50, wherein the variant TCR δ-chain has the CDR3 sequence of a TCR β -chain that is present in the second subset of the second plurality of sequence groups.

Embodiment 52 - The method of embodiment 51, wherein the variant δ-chain does not have the CDR1 sequence and/or CDR2 sequence of the TCR δ -chain that is present in the second subset of the second plurality of sequence groups.

Embodiment 53 -The method according to any one of embodiments 48 to 52, wherein the variant TCR γ-chain is at least about 70%, at least about 80%, at least about 90%, at least about 95%, or at least about 98% identical to the TCR γ-chain that is present in the first subset of the first plurality of sequence groups.

Embodiment 54 - The method according to any one of embodiment 48 to 53, wherein the variant TCR δ -chain is at least about 70%, at least about 80%, at least about 90%, at least about 95%, or at least about 98% identical to the TCR δ-chain that is present in the second subset of the second plurality of sequence groups.

Embodiment 55 -The method according to any one of embodiments 1 to 54, wherein the method further comprises sequencing candidate TCRs that are determined to bind to a peptide of interest in complex with the HLA.

Embodiment 56 - The method according to any one of embodiments 1 to 35, wherein the screening g) comprises in silico modeling of an interaction between respective TCR candidate pairs and the peptide of interest.

Embodiment 57 - A computer system comprising: one or more processors; and a non-transitory computer-readable medium including computer-executable instructions that, when executed by the one or more processors, cause the processors to perform the method according to any one of embodiments 1 to 56.

Embodiment 58 - A non-transitory computer-readable storage medium having stored thereon program code instructions that, when executed by a processor, cause the processor to perform the method according to any one of embodiments 1 to 56.

## Claims

1. A method of identifying a T cell receptor (TCR) that is capable of binding to a peptide of interest in complex with a human leukocyte antigen (HLA), the method comprising:
at a computer system having one or more processors, and memory storing one or more programs for execution by the one or more processors:
a) identifying a first plurality of sequences for a first T-Cell Receptor (TCR) chain and a second plurality of sequences for a second TCR chain from a plurality of sequence read profiles, wherein each respective sequence read profile in the plurality of sequence read profiles comprises corresponding sequence reads for amino acids in a biological sample from a respective subject in a plurality of subjects, and wherein the first TCR chain and second TCR chain are a TCR α-chain and a TCR-β-chain, respectively, or a TCR γ-chain and a TCR δ-chain, respectively;
b) clustering the first plurality of sequences into a first plurality of sequence groups based on sequence similarity between the respective sequences for the first TCR chain in the first plurality of sequences;
c) clustering the second plurality of sequences into a second plurality of sequence groups based on sequence similarity between the respective sequences for the second TCR chain in the second plurality of sequences;
d) identifying a first subset of the first plurality of sequence groups containing sequences that are more prevalent in a first subset of the plurality of subjects that have both the peptide of interest and the HLA than in a second subset of the plurality of subjects that do not have both the peptide of interest and the HLA;
e) identifying a second subset of the second plurality of sequence groups containing sequences that are more prevalent in the first subset of the plurality of subjects that have the peptide of interest and the HLA than in the second subset of the plurality of subjects that do not have the peptide of interest and the HLA;
f) identifying a plurality of TCR candidate pairs, wherein each respective TCR candidate pair comprises a respective sequence for the first TCR chain present in the first subset of the first plurality of sequence groups or a variant thereof and a respective sequence for the second TCR chain present in the second subset of the second plurality of sequence groups or a variant thereof, and
g) screening the plurality of TCR candidate pairs for the ability to bind the peptide of interest, thereby identifying a T cell receptor (TCR) that is capable of binding to a peptide of interest.

2. The method of claim 1, wherein the first TCR chain is a TCR α-chain and the second TCR chain is a TCR β-chain, or wherein the first TCR chain is a TCR γ-chain and the second TCR chain is a TCR δ-chain; and/or wherein the plurality of sequence read profiles comprises at least 500, at least 1,000, at least 5,000, at least 10,000, at least 20,000, at least 50,000, at least 100,000, at least 500,000, at least 1 million, at least 5 million, or at least 10 million sequence read profiles; and/or wherein the corresponding sequence reads for the amino acids in the biological sample from the respective subject are sequence reads from a cancerous tissue of the respective subject.

3. The method according to any one of the preceding claims, wherein the plurality of subjects comprises cancer subjects,
wherein optionally the cancer is selected from urogenital, gynecological, lung, gastrointestinal, head and neck cancer, malignant glioblastoma, malignant mesothelioma, non-metastatic or metastatic breast cancer, malignant melanoma, Merkel Cell Carcinoma or bone and soft tissue sarcomas, hematologic neoplasias, multiple myeloma, acute myelogenous leukemia, chronic myelogenous leukemia, myelodysplastic syndrome and acute lymphoblastic leukemia, non-small cell lung cancer (NSCLC), breast cancer, metastatic colorectal cancers, hormone sensitive or hormone refractory prostate cancer, colorectal cancer, ovarian cancer, hepatocellular cancer, renal cell cancer, pancreatic cancer, gastric cancer, esophageal cancers, hepatocellular cancers, cholangiocellular cancers, head and neck squamous cell cancer soft tissue sarcoma, and small cell lung cancer,
wherein optionally the cancer subjects have undergone a treatment for the cancer,
wherein optionally the treatment is selected from surgery, chemotherapy, immunotherapy, bone marrow transplant, immunotherapy, hormone therapy, targeted drug therapy, cryoablation, radiofrequency ablation, and combinations thereof,
wherein optionally the immunotherapy is selected from an antibody therapy, a cytokine therapy, an oncolytic virus therapy, an adoptive cell transplant therapy, a cancer vaccine or combinations thereof, and
wherein optionally the antibody therapy is a human checkpoint inhibitor therapy, and wherein the human checkpoint is selected from one of the following: PD-1, PD-L1, CTLA-4, LAG3, TIM-3, B7H3, B7H4, A2aR, CD73, NIKG2A, PVRIG/PVRL2, CEACAM1, CECAM 5/6, FAK, CCL2/CCR2, LIF, CD47/SIRPα, CSF-1, IL-1, IL-1R3, IL-8, SEMA4D, Ang-2, CLEVER-1, Axl, and phosphatidylserine,
wherein optionally the peptide of interest is an epitope derived from a tumor-associated antigen, a tumor-specific antigen, or a neoantigen, wherein optionally wherein the peptide of interest is derived from a tumor neoantigen selected from KRAS:p.G12D, BRAF:p.V600E, KRAS:p.G12V, ACVR2A:p.K435fs, GRB14:p.KKK295del, SEC63:p.L532fs, TGFBR2:p.E125fs, ATR:p.K771fs, ICAl:p.N204fs, KRAS:p.G12C, TP53:p.R175H, ABCA8:p.R842Q, ACTL7B:p.R354H, ACVR2A:p.K435fs, AIM2:p.K340fs, ALG2:p.S302Y, ANKIB 1 :p.K144fs, ARSG:p.V131I, ATP10D:p.R311H, AXIN2:p.W663fs, C5orf30:p.D4N, CACNG3:p.V134I, CASP5:p.K78fs, CC2D2A:p.R1284C, CDH10:p.E349K, DNMT1:p.E432K, DOCK2:p.G170R, DOCKS:p.E177K, EGR2:p.R390H, ERBB3:p.V104M, FAM135B:p.R884H, FBXW7:p.R505C, FBXW7:p.R465H, FHDC1:p.R254W, FOXL1:p.N89K, HCN4:p.R525H, HLA-DMA:p.E84K, HTR3B:p.R236C, ITGA4:p.T673M, KIF18A:p.R17C, KIF20B:p.E991K, KLHL5:p.R326C, KRAS:p.A146T, KRAS:p.G13D, LPHN3:p.R1183Q, MAP2K4:p.R287H, MAPK8IP1:p.L217fs, MFSD5:p.R280Q, MUC16:p.R8606H, MYO6:p.D1180N, NAA25:p.S807Y, NBPF14:p.V44L, NRAS:p.Q61K, NRAS:p.G13R, PAX3:p.T424M, PGAM1:p.R240H, PHF3:p.R1410I, PIK3CA:p.R88Q, PIK3CA:p.E545K, PIK3CA:p.H1047R, PLXNA3:p.V14fs, POSTN:p.R508C, PTPRU:p.D1434N, PYGO2:p.Q150fs, RBBP7:p.E274K, SFPQ:p.R611Q, SGSM1:p.F1117L, SLC25A40:p.R96Q, SLC8A1:p.R431H, SLITRK3:p.S298L, SPATA22:p.S150L, SUN3:p.E128K, TGFBR1:p.S241L, TP53:p.R273H, TP53:p.R273C, TP53:p.R248W, TRPVS:p.R492H, USP40:p.S851L, VPS13C:p.D1359Y, ZBTB24:p.L607I, ZNF434:p.R306C, ZNF443:p.R301I, ZNF484:p.R138C, and ZNF770:p.S441P, and wherein optionally the tumor neoantigen and HLA allele are selected from the following neoantigen and HLA allele pairs: TP53 (R175H)-A*02:01, TP53 (Y220C)-A*02:01, and TP53 (R248W)-A*68:01,
wherein optionally the plurality of subjects do not exhibit tumor cell loss of heterozygosity.

4. The method according to claim 1 or claim 2 wherein the plurality of subjects comprises subjects having a viral infection, and wherein the peptide of interest is an epitope derived from an antigen of the virus.

5. The method according to any one of claims 1 to 4, wherein the clustering of the first plurality of sequences and/or the clustering of the second plurality of sequences is based on at least CDR3 sequence similarity; or
wherein the clustering of the first plurality of sequences and/or the clustering of the second plurality of sequences is based on at least CDR3 length; or
wherein the clustering of the first plurality of sequences and/or the clustering of the second plurality of sequences is based on identical sequence length and sequence similarity; and/or
wherein the clustering of the first plurality of sequences and/or the clustering of the second plurality of sequence is performed on at least 1 × 10², at least 1 × 10³, at least 1 × 10⁴, at least 1 × 10⁵, or at least 1 × 10⁶ sequences.

6. The method according to any one of the preceding claims, wherein the respective sequences for the first TCR chain in the first plurality of sequence groups and the respective sequences for the second TCR chain in the second plurality of sequence groups are collectively representative of TCR sequences in the corresponding biological samples for at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95% or at least 99% of the plurality of subjects.

7. The method according to any one of the preceding claims, wherein the first subset of the first plurality of sequence groups contains TCR α-chain sequences and the second subset of the second plurality of sequence groups contains TCR β-chain sequences; or wherein the first subset of the first plurality of sequence groups contains TCR γ-chain sequences and the second subset of the second plurality of sequence groups contains TCR δ-chain sequences.

8. The method according to any one of the preceding claims, wherein sequences of the first plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects, and/or wherein sequences of the second plurality of sequence groups are identified as more prevalent in the first subjects of the plurality of subjects than in the second subset of the plurality of subjects if the sequences are present above a first threshold percentage in the first subjects of the plurality of subjects and below a second threshold percentage in the second subset of the plurality of subjects, wherein optionally the first threshold percentage is about 0.1%, about 0.5%, about 1%, about 5%, about 10%, about 15%, about 20%, about 25%, about 30%, about 35%, about 40%, about 45%, about 50%, about 60%, about 65%, about 70%, about 75%, about 80%, about 85%, about 90%, about 95% or about 99% and/or wherein the second threshold percentage is about 0.5%, about 1%, about 5%, about 10%, about 15%, about 20%, about 25%, about 30%, about 35%, about 40%, about 45%, or about 50%.

9. The method according to any one of the preceding claims, wherein respective subjects in the first subset of the plurality of subjects and the second subset of the plurality of subjects are identified based on sequencing data from corresponding samples of cancerous tissues from the respective subjects.

10. The method according to any one of the preceding claims, wherein the identifying f) comprises screening for TCR pairs in which the respective sequence for the first TCR chain and the respective sequence for the second TCR chain are co-expressed in a threshold number of subjects in the first subset of the plurality of subjects, and wherein optionally the threshold number of subjects is at least 10, at least 25, at least 50, or at least 100 subjects.

11. The method of any one of the proceeding claims, wherein the respective sequence for the first TCR chain comprises a CDR3 sequence of the first TCR chain and the respective sequence for the second TCR chain comprises a CDR3 sequence of the second TCR chain.

12. The method of any one of the proceeding claims, wherein each respective TCR candidate pair comprises a first TCR chain and a second TCR chain that is expressed in the same subject, wherein optionally the first TCR chain is a TCR α-chain and the second TCR chain is a TCR β-chain, or wherein the first TCR chain is a TCR γ-chain and the second TCR chain is a TCR δ-chain.

13. The method of any one of the proceeding claims, wherein the screening step g) comprises expressing the plurality of TCR candidate pairs in a plurality of cells, wherein an individual cell of the plurality of cells express a TCR candidate pair of the plurality of TCR candidate pairs, wherein optionally the screening step g) comprises contacting the plurality of cells expressing the plurality of TCR candidate pairs with the peptide of interest/MHC (pMHC) multimers and isolating cells that bind the pMHC multimers, wherein optionally the cells that bind the pMHC multimers are isolated using a cell sorting method, wherein optionally the cell sorting method is selected from magnetic-activated cell sorting (MACS), fluorescence-activated cell sorting (FACS), and buoyancy-activated cell sorting, and wherein optionally the pMHC multimers are tetramers.

14. The method of any one of the preceding claims, wherein one or more TCR candidate pairs in the plurality of TCR candidate pairs comprises:
a) a variant of a TCR α-chain that is present in the first subset of the first plurality of sequence groups; and/or
b) variant of a first TCR β -chain that is present in the second subset of the second plurality of sequence groups,
wherein optionally the variant TCR α-chain has the CDR3 sequence of a TCR α-chain that is present in the first subset of the first plurality of sequence groups, wherein optionally wherein the variant TCR α-chain does not have the CDR1 sequence and/or CDR2 sequence of the TCR α-chain that is present in the first subset of the first plurality of sequence groups, wherein optionally the variant TCR β -chain has the CDR3 sequence of a TCR β -chain that is present in the second subset of the second plurality of sequence groups, wherein optionally the variant β-chain does not have the CDR1 sequence and/or CDR2 sequence of the TCR β -chain that is present in the second subset of the second plurality of sequence groups, wherein optionally the variant TCR α-chain is at least about 70%, at least about 80%, at least about 90%, at least about 95%, or at least about 98% identical to the TCR α-chain that is present in the first subset of the first plurality of sequence groups, wherein optionally the variant TCR β-chain is at least about 70%, at least about 80%, at least about 90%, at least about 95%, or at least about 98% identical to the TCR β-chain that is present in the second subset of the second plurality of sequence groups.

15. The method of any one of claims 1 to 14, wherein one or more TCR candidate pairs of the plurality of TCR candidate pairs comprises:
a) a variant of a TCR γ-chain that is present in the first subset of the first plurality of sequence groups; and/or
b) variant of a first TCR δ -chain that is present in the second subset of the second plurality of sequence groups,
wherein optionally the variant TCR γ-chain has the CDR3 sequence of a TCR α-chain that is present in the first subset of the first plurality of sequence groups, wherein optionally the variant TCR γ-chain does not have the CDR1 sequence and/or CDR2 sequence of the TCR γ-chain that is present in the first subset of the first plurality of sequence groups, wherein optionally the variant TCR δ-chain has the CDR3 sequence of a TCR β -chain that is present in the second subset of the second plurality of sequence groups, wherein optionally the variant δ-chain does not have the CDR1 sequence and/or CDR2 sequence of the TCR δ -chain that is present in the second subset of the second plurality of sequence groups, wherein optionally the variant TCR γ-chain is at least about 70%, at least about 80%, at least about 90%, at least about 95%, or at least about 98% identical to the TCR γ-chain that is present in the first subset of the first plurality of sequence groups, wherein optionally the variant TCR δ -chain is at least about 70%, at least about 80%, at least about 90%, at least about 95%, or at least about 98% identical to the TCR δ-chain that is present in the second subset of the second plurality of sequence groups.

16. The method of any one of the proceeding claims, wherein the method further comprises sequencing candidate TCRs that are determined to bind to the peptide of interest in complex with the HLA, and/or wherein the screening g) comprises in silico modeling of an interaction between respective TCR candidate pairs and the peptide of interest.

17. A computer system comprising:
one or more processors; and
a non-transitory computer-readable medium including computer-executable instructions that, when executed by the one or more processors, cause the processors to perform the method according to any one of claims 1-16.

18. A non-transitory computer-readable storage medium having stored thereon program code instructions that, when executed by a processor, cause the processor to perform the method according to any one of claims 1-16.
